# EUROPEAN PATENT APPLICATION

(11) **EP 1 709 959 A1**
(43) Date of publication of application: **11.10.2006**
(21) Application number: 05703441.5
(22) Date of filing: 11.01.2005
(51) Int. Cl.: A61K 31/095, A61K 49/00, A61P 27/02, A61B 3/10, G01N 33/487

(54) **REAGENT FOR LACRIMATION EXAMINATION AND METHOD OF LACRIMATION EXAMINATION**

(30) Priority: 16.01.2004 JP 2004008808; 08.11.2004 JP 2004324327
(71) Applicant: HOUSE FOODS CORPORATION, Higashi-Osaka-shi, Osaka 577-0036 (JP); Kinoshita, Shigeru, Osaka-shi Osaka 5450035 (JP); Yokoi, Norihiko, Kita-ku, Kyoto-shi, Kyoto 6038147 (JP); Higashihara, Hisayo, Kyoto-shi, Kyoto 6020802 (JP)
(72) Inventor: KINOSHITA, Shigeru, 5450035 (JP); YOKOI, Norihiko, Kita-ku, Kyoto 6038147 (JP); HIGASHIHARA, Hisayo, Kamigyo-ku, Kyoto 6020802 (JP); SHIOMI, Nobuo, c/o House Foods Corporation, Higashi-Osaka-shi, Osaka 5770036 (JP); IMAI, Shinsuke, c/o House Foods Corporation, Higashi-Osaka-shi, Osaka 5770036 (JP); TSUGE, Nobuaki, c/o House Foods Corporation, Higashi-Osaka-shi, Osaka 5770036 (JP); HORIE, Kentaro, 3660034 (JP)
(74) Representative: Müller Fottner Steinecke
(86) International application number: PCT/JP2005/000202
(87) International publication number: WO 2005/067907

(57) **Abstract**

A new application of the dacryogenic component of onions and analogues of the component in the medicinal field. It is a reagent for lacrimation examination which contains as an active ingredient a compound represented by the following chemical formula: 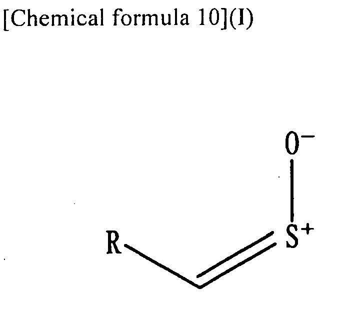 wherein R represents a C₁₋₅ alkyl chain.

## Description

### TECHNICAL FIELD

The present invention relates to a use of a composition having an effect of promoting lacrimation. More specifically, the present invention relates to a reagent for lacrimation examination and a method of lacrimation examination, as well as a lacrimation promoter composition. The present invention is suitably used for diagnosis and treatment of dry eye.

### BACKGROUND ART

The lacrimatory property of onions is well known. The onion cells are rich in PRENCSO, a sulfur-containing amino acid. A lacrimatory component (Thiopropanal S-Oxide) is generated from PRENCSO by alliinase and lacrimatory factor synthase (LFS), and thereby lacrimatory property of onions is exhibited (Nature 419, 685, 2002: non-patent document 1, JP Patent No. 330305: patent document 1). Since thiopropanal S-Oxide is volatile, it is presumed that the lacrimatory component stimulates the mucosa of the eye or the nose during processing of onions, thereby allowing the reflex lacrimation to be induced from the lacrimal gland. Furthermore, it is thought that the mechanism of this lacrimation is carried out via a reflective passage of lacrimation known as a reflex loop, that is, the nerve system including sensory nerve - brain stem - parasympathetic nerve - lacrimal gland. Tears (lacrimal fluids) form a refractive film on the ocular surface, protect the epithelial cells of the ocular surface from infection, and supply the epithelial cells with nutrients and oxygen. Decrease in lacrimation causes dry eye. The deficiency in tears makes the epithelial cells dry so as to be desquamated, thus causing the epithelium disorder and various eye symptoms. According to a large scale epidemiological study carried out by the present inventors, the number of dry eye patients is estimated to be as many as 22 million.
While the lacrimatory property of onions is regarded as an unwanted side effect in the area of food processing, it can be a research theme of great potential in the area of physiology of tears and the application study thereof.

Patent document 1: JP Patent No. 3330305
Non-patent document 1: Nature 419, 685, 2002

### DISCLOSURE OF INVENTION

### [Problems to be Solved by the Invention]

With the background stated above, an object of the present invention is to provide a new application of a lacrimatory component of onions and the analogous compounds thereof in the medical field.

### [Means for Solving the Problems]

With the above-mentioned object, the present inventors have examined whether or not the lacrimatory component of onions can be used in lacrimation examinations and can be used as a therapeutic drug for ocular surface diseases. Firstly, in normal subjects, the effect of a lacrimatory component of onions on the lacrimation was examined. As a result, the following findings were obtained. (1) The tear volume increased within a short time and the change in the tear volume was transient. This result suggests that the lacrimatory component is effective as a stimulant of lacrimation and that the use thereof does not produce any adverse side effects. Further, this result means that when the lacrimatory component is used for lacrimation examination, measurement can be completed in a short time. (2) When one of the eyes of a subject was exposed to the lacrimatory component, lacrimation was induced in the other eye that was not exposed (non-exposed eye). This means that the lacrimatory component has a high lacrimation inducing potential and that when it is used as a therapeutic drug, high therapeutic effect can be obtained, and the degree of freedom of administration mode is increased.
After the above-mentioned findings were obtained, furthermore, in dry eye patients, the effects of the lacrimatory component of onions on the lacrimation and the dynamics thereof were examined. As a result, the following findings were obtained. (3) Also in dry eye patients, the lacrimation was promoted by stimulation of the lacrimatory component. This result indicates the probability that the lacrimatory component can be used as a therapeutic drug for dry eye. (4) Difference in time taken before the stimulation of the lacrimatory component was felt and in the degree of stimulation felt by the subject was found between normal subjects and dry eye patients. Therefore, it is suggested that the stimulation of the lacrimatory component can be used as an index in diagnosis of dry eye. (5) Difference in the dynamics of the lacrimation by the stimulation of the lacrimatory component was found between normal subjects and dry eye patients. Furthermore, difference in the dynamics of the lacrimation was found between a slight / moderate group of dry eye patients and a severe group of dry eye patients. These findings mean that the use of the amount of lacrimation induced by the stimulation of the lacrimatory component as an index allows not only diagnosis of dry eye but also the determination of the severity of dry eye conditions. (6) In both normal subjects and dry eye patients, the tear volume returned to the initial volume within a short time after the stimulation of the lacrimatory component had been given. Thus, it is suggested that, not only in normal subjects, but also in dry eye patients, the effect of the lacrimatory component is transient and that the adverse effects to the ocular surface does not occur. (7) Although the average degree of stimulation from the lacrimatory component reported by each subject was different between normal subjects and dry eye patients, both returned to zero (i.e., no stimulation) at the end of the examination, and no effect on the epithelium of the ocular surface was observed after the examination. These results indicate that there are no substantial adverse effects by the stimulation of the lacrimatory component on the ocular surface. Therefore, when the lacrimatory component is used as a reagent for examining dry eye or as a therapeutic drug thereof, there is little chance for any side effects (adverse effects) to occur, and that the lacrimatory component can be used repeatedly for an extended time.

To date, as a lacrimation examination, the Schirmer test has been widely used. The quantitative examination of tears may be classified into either a measurement of the amount of lacrimation induced by stimulation on the ocular surface or a measurement of the tear volume without stimulation. A representative example of the former measurement is the Schirmer test. In the Schirmer test, the amount of tears is measured with a strip of filter paper inserted under the eyelid for 5 minutes. In general, when the length (distance) of the filter paper wet with tears is not less than 10 mm, the subject is diagnosed to be normal. On the other hand, when the length is not more than 5 mm, the subject is diagnosed to have a dry eye. Because of its invasiveness, the Schirmer test is a very stressful examination for dry eye patients who have epithelial abnormalities on the ocular surface. Nevertheless, since no alternative effective examination method has been developed, the Schirmer test has been used for more than 100 years.
The above findings by the present inventors demonstrates that the lacrimatory component of onions can be preferably used as a stimulant (examination reagent) in the lacrimation examination. A lacrimatory component is volatile and the stimulation thereby may be given in a non-contact manner, so that the lacrimation examination method using a lacrimatory component is little invasive. Thus, the inventors' investigation demonstrated that the use of the lacrimatory component of onions allows less invasive lacrimation examinations than the existing Schirmer test.
On the other hand, it was demonstrated that the lacrimatory component of onions showed the lacrimation effect on not only normal subjects but also dry eye patients having epithelial abnormalities on the ocular surface. This means that the lacrimatory component of onions can be used as a therapeutic drug for treating dry eye. Furthermore, since the lacrimatory component of onions has a high lacrimation effect, the lacrimatory component of the present invention may be used for treatment of not only dry eye but also various disorders, for which promotion of lacrimation may have therapeutic effects.

The present invention has been accomplished based on the above-mentioned research results and is comprised of the following components.
[1] A reagent for lacrimation examination, comprising as an active ingredient a compound represented by the following chemical formula: 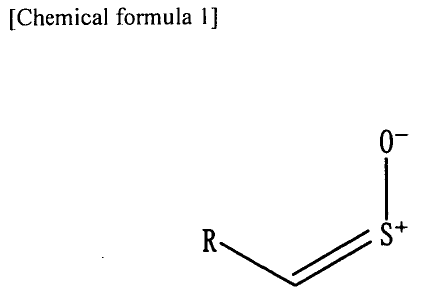 wherein R represents a C₁₋₅ alkyl chain.
[2] A reagent for lacrimation examination, comprising as an active ingredient a compound represented by the following chemical formula. 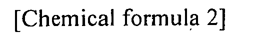 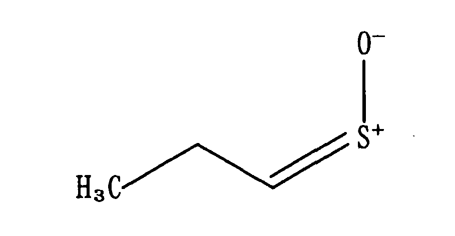
[3] The reagent for lacrimation examination described in [1] or [2], which is used for examining dry eye (diagnosis of dry eye).
[4] A lacrimation examination kit, comprising:
   a reagent for lacrimation examination described in any of [1] to [3]; and
   an exposure container used for exposing the eye to the reagent for lacrimation examination, the exposure container being used to cover the eye in a state in which the inner side thereof faces the eye after the reagent for lacrimation examination is placed in the exposure container.
[5] A method of lacrimation examination, the method comprising:
   exposing an eye of a subject to the reagent for lacrimation examination described in any of [1] to [3].
[6] A method of lacrimation examination, the method comprising:
   an exposure step of exposing an eye of a subject to the reagent for lacrimation examination described in any of [1] to [3]; and
   a stimulation measurement step of measuring a length of time from the time when the exposure step is started to the time when the subject feels the stimulation, or an degree of stimulation felt by the subject at a certain time point after the start of the exposure step.
[7] A method of lacrimation examination, the method comprising:
   an initial tear volume measurement step of measuring a tear volume of a subject in an initial state (the initial tear volume);
   an exposure step of exposing an eye of the subject to the reagent for lacrimation examination described in any of [1] to [3];
   a tear volume measurement step of measuring a tear volume of the subject at a certain time point after the exposure step (a post-exposure tear volume) and/or monitoring an increase with time of the tear volume until that time point; and optionally,
   a calculation step of calculating the difference in the tear volume in the subject between the initial tear volume and the post-exposure tear volume.
[8] A method of lacrimation examination, the method comprising:
   an exposure step of exposing an eye of a subject to the reagent for lacrimation examination described in any of [1] to [3]; and
   a tear volume measurement step of measuring a tear volume of the subject at a certain time point after the exposure step.
[9] The method of lacrimation examination described in [7] or [8], wherein the tear volume is measured using a radius of tear meniscus curvature.
[10] The method of lacrimation examination described in any of [5] to [9], wherein the exposure of the reagent for lacrimation examination to the eye of the subject is accomplished in a non-contact manner.
[11] A lacrimation promoter composition, containing as an active ingredient a compound represented by the following chemical formula: 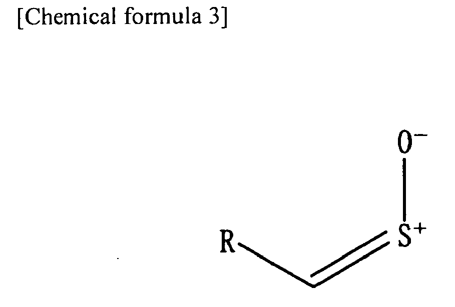 wherein R represents a C₁₋₅ alkyl chain.
[12] A lacrimation promoter composition, containing as an active ingredient a compound represented by the following chemical formula. 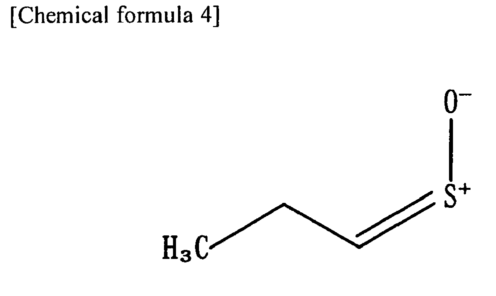
[13] The lacrimation promoter composition described in [11] or [12], which is used for prevention or treatment of dry eye.
[14] A lacrimation promoter kit, comprising:
   the lacrimation promoter composition described in any of [11] to [13]; and
   an exposure container used for exposing an eye to the reagent for lacrimation examination, wherein the exposure container is used to cover the eye in a state in which the inner side thereof faces the eye after the lacrimation promoter composition is placed in the exposure container.

### [Effect of the Invention]

The reagent for lacrimation examination of the present invention can promote lacrimation of a subject by stimulation given in a non-contact manner. Therefore, a lacrimation examination using thereof is less invasive and less burdensome (painful) to the subject compared to the currently used examination methods.

### BRIEF DESCRIPTION OF DRAWINGS

Fig. 1 is a table showing Thiopropanal S-Oxide, a lacrimatory component of onions, and the analogues thereof.
Fig. 2 is a summary of synthetic reactions for a lacrimatory component (Thiopropanal S-Oxide).
Fig. 3 is a synthesis scheme for an intermediate product (1-propanesulfinyl chloride) used in the synthesis of a lacrimatory component (Thiopropanal S-Oxide).
Fig. 4 is a scheme for synthesizing a lacrimatory component (Thiopropanal S-Oxide) from the intermediate product (1-propanesulfinyl chloride).
Fig. 5 shows an exposure container I used in Examples of the present invention. Fig. 5a is a perspective view of an exposure container 1 in open state; and Fig. 5b shows a detailed configuration of the exposure container 1 in closed state.
Fig. 6 shows a basic configuration of a video meniscometer used in Examples of the present invention.
Fig. 7 is a graph showing the effects of exposing the ocular surface of an eye to ether (left column); and the effects of a lacrimatory component delivered via nasal passage (right column).
Fig. 8 is a graph summarizing the measurement results of the length of time from the exposure of a lacrimatory component to the time of the stimulation first felt by the subject (ST).
Fig. 9 is a graph comparing the degrees of stimulation induced by the exposure of a lacrimatory component.
Fig. 10 is a graph showing the change with time of the degrees of stimulation induced by the exposure of a lacrimatory component.
Fig. 11 is a graph comparing the increase of the tear volume two minutes after the start of the exposure of eyes to a lacrimatory component.
Fig. 12 is a graph summarizing the relationship between the exposure time of a lacrimatory component and the increase of the tear volume.
Fig. 13 is a graph summarizing the measurement results of time from the start of the exposure of a lacrimatory component to the time of the stimulation first felt (ST) in dry eye patients and normal subjects.
Fig. 14 is a graph comparing the degrees of stimulation induced by the exposure of a lacrimatory component between dry eye patients and normal subjects. This graph shows the comparison of the degrees of stimulation in normal subjects, a slight / moderate patient group, and a severe patient group at the longest exposure time.
Fig. 15 is a graph comparing the transition of the degrees of stimulation induced by the exposure of a lacrimatory component between dry eye patients and normal subjects.
Fig. 16 is a graph comparing the increase in the tear volume two minutes after the start of the exposure of a lacrimatory component between dry eye patients and normal subjects.
Fig. 17 is a graph comparing the increase with time of the tear volume between dry eye patients and normal subjects.
Fig. 18 is a graph illustrating a relationship between the radius of tear meniscus curvature (R) and extensibility (Grade) of the tear lipid layer.
Fig. 19 is a graph depicting the change of the radius of tear meniscus curvature (R) due to the exposure of a lacrimatory component.
Fig. 20 is a graph depicting the change of the extensibility (Grade) of the tear lipid layer due to the exposure of a lacrimatory component.
Fig. 21 shows the relationship between the change of the radius of tear meniscus curvature (R) and the change of the extensibility (Grade) of the tear lipid layer due to the exposure of a lacrimatory component before and after the exposure.
Fig. 22 shows a graph depicting the relationship between the amount of change (ΔR) in the radius of tear meniscus curvature (R) due to the exposure of a lacrimatory component and the amount of change (ΔGrade) of the extensibility (Grade) of the tear lipid layer.
Fig. 23 is a graph depicting the change of the radius of tear meniscus curvature (R) due to the exposure of a lacrimatory component in the eye with a soft contact lens.
Fig. 24 is a graph depicting the change of the specular image of tear on a soft contact lens by exposure of a lacrimatory component.
Fig. 25 shows the change of NIBUT of tear on a soft contact lens by exposure of a lacrimatory component.
Fig. 26 shows some representative examples of the radius of tear meniscus curvature (R) before and after the exposure of a lacrimatory component.
Fig. 27 shows a representative example of the specular images of tear on a soft contact lens (the same examples, before and after the exposure of a lacrimatory component).
Fig. 28 shows some representative examples of NIBUT of tear on a soft contact lens (the same examples, before and after the exposure of a lacrimatory component).
Fig. 29 shows a method for evaluating the thickness and stability of tear on a soft contact lens.

### [Discription of Notations]

1 exposure container
11 outer container main body
12 lid of outer container
20 inner container

### BEST MODE FOR CARRYING OUT THE INVENTION

One aspect of the present invention relates to a reagent for lacrimation examination. In the present invention, the "reagent for lacrimation examination" denotes a reagent having an effect of promoting lacrimation in a subject (test subject) to whom the reagent is applied and, in principle, the reagent is used for examination based on the amount of tears as an index. However, the term "reagent for lacrimation examination" is also used in a broader sense that include a reagent used for an examination based on such indices as a time taken for the subject to feel the stimulation after application thereof or an degree of the stimulation, and so forth. The reagent for lacrimation examination is typically used for examinations (diagnosis) of dry eye. The "dry eye" herein is defined as "a disorder of cornea and conjunctiva epithelium, which is caused by qualitative and quantitative abnormalities of the tear (film)" (Jun Shimazaki: Definition and criteria of dry eye. Ophthalmology 37: 765-770, 1995) . Dry eyes are generally classified into two types: a tear deficiency type dry eye and an excessive tear evaporation type dry eye (Lemp M:Report of the National Eye Institute/Industry workshop on Clinical Trials in Dry Eyes. CLAO J 21:221-232, 1995). The former includes Sjögren syndrome or a tear deficiency type dry eye other than Sjögren syndrome. The latter includes Meibomian gland dysfunction, blinking dysfunction or decrease of blinking due to various causes [including dry eyes related to VDT (Visual display terminal)], wearing of contact lens, severe ocular surface diseases (Stevens-Johnson syndrome, ocular cicatricial pemphigoid, and the like). In this patent specification, however, the term "dry eye" is used in a wider sense including symptoms such as dry eye related to conjunctivochalasis and allergic diseases of conjunctiva (dryness, congestion, increase in discharge, lacrimation, and the like); and symptoms like dry eye of postoperative eye (after corneal transplantation, after refractive operation, after cataract operation, and the like).

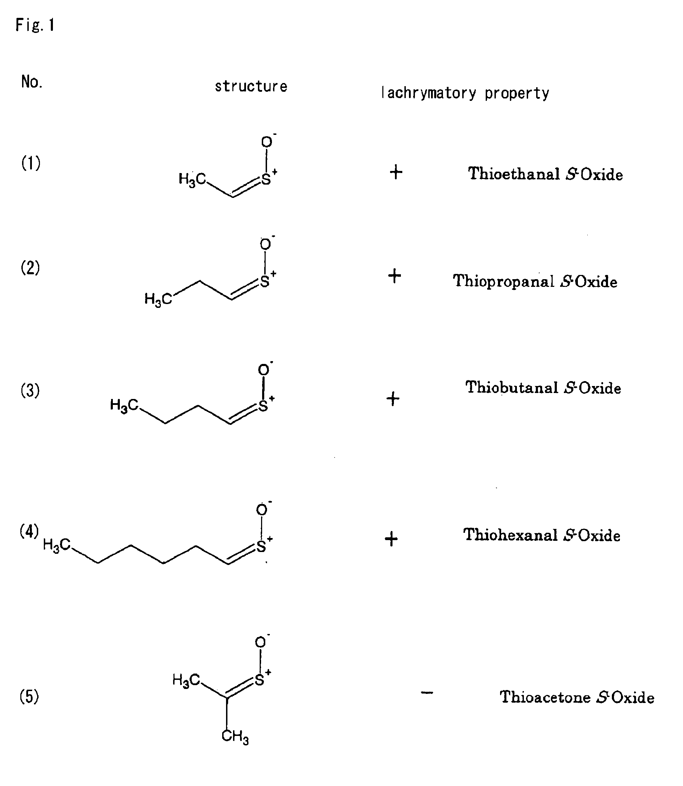

The reagent for lacrimation examination of the present invention is characterized by those that include as an active ingredient a compound represented by the following chemical formula: 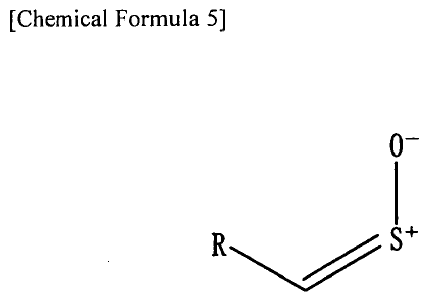 wherein R represents a C₁₋₅ alkyl chain.
The above-mentioned chemical formula is Thiopropanal S-Oxide (R=C₂), that is a lacrimatory component of onions, and the analogues thereof. Thiopropanal S-Oxide is represented by the following chemical formula. 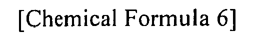 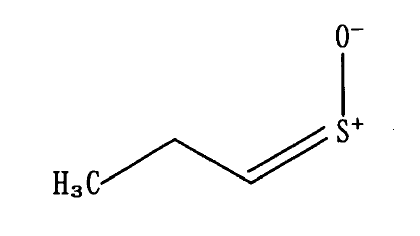 As shown in Fig. 1, Thiopropanal S-Oxide, which is a lacrimatory component of onions, has a structure called Thioalkanal S-Oxide. It is reported that among these analogues shown in Fig. 1, Thioethanal S-Oxide (1), Thiobutanal S-Oxide (3), and Thiohexanal S-Oxide (4) have the lacrimatory property similar to Thiopropanal S-Oxide (J. Agr. Food Chem. 19, 269-272 (1971)).
Thiopropanal S-Oxide and the analogues thereof can be prepared through extraction and purification from natural sources. Otherwise, these compounds may be prepared by chemical syntheses. Thiopropanal S-Oxide and the analogues thereof can be prepared with reference to J. Agr. Food Chem. 19, 269-272 (1971), J. Am. Chem. Soc. Vol. 118, No.32, 1996, 7492-7501 and JP Patent No. 3330305.

Depending on the preparation methods and the form of use there of, the reagent for lacrimation examination of the present invention may be provided in a form of an ethyl ether solution or the like. The appropriate amount of an active ingredient in the reagent for lacrimation examination of the present invention is an amount capable of obtaining the intended effects (that is, an amount with which an effective examination can be performed. For example, in the case of examination (or diagnosis) of dry eye, the amount means an amount sufficient for the diagnosis of dry eye, or determination of severity of the dry eye condition). The amount can be determined taking into consideration such factors as the kind of active ingredients or the amount for a single dose. When Thiopropanal S-Oxide is used as the active ingredient, for example, an appropriate single dose of the active ingredient can be determined as follows (see also Examples below).
Firstly, Thiopropanal S-Oxide is synthesized in accordance with the method described in "J. Am. Chem. Soc. Vol. 118, No. 32, 1996, 7492-7501" to prepare an ethyl ether solution. The amount of Thiopropanal S-Oxide in the solution is determined as follows: 1 µl of ethyl ether solution is subjected to HPLC under the following conditions and the peak area of Thiopropanal S-Oxide (hereinafter, referred to as an "area value") is measured. This area value is used as a provisional measure of the amount of Thiopropanal S-Oxide in 1 µl of ethyl ether solution.

| (HPLC conditions) | |
|---|---|
| Instrument used : | SHIMADZU 10A Series |
| Ultraviolet and visible light detector : | SHIMADZU SPD-10AV |
| Chromatographic pack : | SHIMADZU CR-5A |
| Column : | Pegasil ODS (Senshu Scientific co., ltd) |
| Solvent: | 30% MeOH (pH 3.3, TFA) |
| Flow rate : | 0.6 ml/min |
| Column temperature : | 35°C |
| Detection wavelength : | 210 nm |

A single dose of a reagent for lacrimation examination of the present invention may contain Thiopropanal S-Oxide in the amount corresponding to 3 µl or more of the ethyl ether solution whose area value measured as mentioned above is about 1,700,000.
Note here that the proper amount of active ingredients other than Thiopropanal S-Oxide can also be determined in a similar manner as described above.

In one embodiment of the present invention, the above-mentioned reagent for lacrimation examination is provided as a part of an eye examination kit. The kit consists of a dedicated container (exposure container) used for exposing a subject's eye to the reagent for lacrimation. The container is used in the following manner: a predetermined amount of the reagent for lacrimation examination is placed in the container; and then the container is used for covering an eye with the inner side thereof facing the eye (as a specific example, see an exposure container described in the Example mentioned below). By using such a container, the eye to be tested can be exposed to the active ingredient in the reagent for lacrimation examination in a non-contact manner.

Another aspect of the present invention relates to a method of use of the above-mentioned reagent for lacrimation examination. Specifically, the present invention provides a lacrimation examination method. The lacrimation examination method of the present invention includes at least an exposure step of exposing the eye of a subject (test subject) to the above-mentioned reagent for lacrimation examination. It is preferable that the exposure of the reagent for lacrimation examination to the eye of the subject is accomplished in a non-contact manner. Exposure in a non-contact manner gives no direct physical stimulation to the eye. Thus, the examination can be less stressful (burdensome) to the subject (test subject). The exposure in a non-contact manner can be achieved using, for example, the above-mentioned exposure container in the following manner. Firstly, a predetermined amount of the reagent for lacrimation examination is added to the exposure container (it is preferable to add the reagent on a filter paper, or the like, placed inside the exposure container, so that the reagent for lacrimation examination may not run off and stay inside the container). Thereafter, the exposure container is allowed to cover an eye with the inner side thereof facing the eye. In order to carry out the effective exposure, the shape of the open mouth of the exposure container is preferably designed so that it fits well with the curveture of the periphery around the eye to ensure adequate sealing (it is preferable to use an elastic material such as silicone rubber for constructing the mouth of the exposure container).

In one embodiment of the present invention, after the exposure step, "a stimulation measurement step of measuring the length of time from the time when the exposure step is started to the time when the subject (test subject) feels the stimulation (denoted as stimulation time, ST), or the degree of stimulation felt by the subject (test subject) at a certain time point after the start of the exposure step.
In this embodiment, the stimulation time (ST) or the degree of stimulation are examination indices. As shown in the Examples described below, ST of dry eye patients tends to be longer than that of normal subjects. Furthermore, when the stimulation starts to be felt or at longest exposure time, the degree of stimulation tends to be low. Therefore, when the ST of the subject is longer than the predetermined normal range of ST (ST of normal subject), and similarly, when the degrees of stimulation felt by the subject is lower than than that in the predetermined normal range (the degrees of stimulation of normal subject), it can be determined that the eye is dry eye. Furthermore, by classifying the ST or the degree of stimulation according to the severity of dry eye conditions, the severity of other subjects can be determined on the basis of the measured ST or the degree of stimulation.
ST and the degree of stimulation are subjective, for they are typically measured based on self declaration by the subject. However, a more objective measure, such as the time taken for the subject to start blinking induced by the stimulation or the length of time beyond which the subject no longer can endure the stimulation, or the like, can be used as an alternative to ST.

In another embodiment of the present invention, first, "an initial tear volume measurement step of measuring a tear volume of a subject before the exposure step, (denoted as initial tear volume)" is carried out. Next, "a tear volume measurement step of measuring a tear volume of the subject some predetermined time after the start of exposure (post-exposure tear volume) and/or an increase of the tear volume. over time up to a certain point in time during exposure is carried out. Thereafter, optionally, a calculation step of calculating the tear volume increase from the initial tear volume to the post-exposure tear volume.
In this embodiment, the increase of the tear volume (tear volume increase) serves as an index for the eye examination. As shown in the Examples described below, the tear volume increase in dry eye patients is smaller than that in normal subjects. Moreover, the higher the degree of severity becomes, the more pronounced the difference from normal subjects in the tear volume increase tends to be. Therefore, it can be determined that the eye is dry eye when the tear volume increase is smaller than a predetermined tear volume increase for normal subjects. Furthermore, by classifying the tear volume increase according to the severity of dry eyes, the degree of severity of dry eyes can be determined based on the newly measured tear volume increase.
Note here that the tear volume measurement step can be carried out, for example, 1, 2, 3, 4, 5, 6, 7, 8, 9, 10, 11, 12, 13, 14 and 15 minutes after the start of the exposure step.

In a further embodiment of the present invention, at a certain time point after the start of the exposure step, "a tear volume measurement step of measuring a tear volume of the subject" is performed and based on the measured tear volume the examination results are determined. As shown in the Examples described below, in dry eye patients, the post-exposure tear volumes are generally smaller than those in normal subjects. Moreover, as the degree of severity becomes higher, the difference in the tear volume between dry eye patients and normal subjects tends to become more pronounced. Therefore, it can be determined that the subject has a dry eye if the measured tear volume is smaller than the predetermined normal range for the normal subjects. Furthermore, by classifying the tear volume according to the degree of severity of dry eyes in advance, the degree of severity of an dry eye can be determined based on the tear volume measured for that eye.

The measured tear volume obtained in the tear volume measurement step can also be used for monitoring the degree of severity of dry eye. Specifically, the measured tear volume is compared with the tear volume that was measured previously. If it is found that the tear volume is decreasing, it is determined that the condition is deteriorating. When it is found that the tear volume is increasing, it is determined that the condition is improving. Thus, the deterioration (or improvement) of dry eyes can be monitored using the tear volume as an index. Similarly, not by using the tear volume per se, but by using the tear volume increased as a result of the exposure step, (that is to say, the tear volume increase), the degree of severity of dry eyes can be monitored. In this case, specifically, the examination is carried out by the following procedure.
First, an initial tear volume measurement step of measuring a tear volume of the subject before an exposure (an initial tear volume) is carried out. Next, an exposure step of exposing an eye of a subject to the reagent for lacrimation examination is performed. Subsequently, at a certain time point after the start of exposure step, a tear volume measurement step of measuring the tear volume of the subject (post-exposure tear volume) is carried out. After the above-mentioned steps, using the initial tear volume and the post-exposure tear volume, a tear volume increase calculation step of calculating the tear volume increase in the subject is carried out. Next, a determination step of determining that the condition has deteriorated when it is found that the tear volume increase decreased from the previously measured value and that the condition has improved when it is found that the tear volume increase increased from the previously measured value.

The methods of measuring the tear volume in the above-mentioned embodiments are not particularly limited. However, it is preferable to employ a method based on the radius of tear meniscus curvature (meniscometry, video meniscometry, and the like) (see, for example, Yokoi N, et al.: Reflective meniscometry: a non-invasive method to measure tear meniscus curvature. Br J Ophthalmol, 83, 92-97, 1999; Yokoi N, et al.: Reflective meniscometry: a new field of dry eye assessment. Cornea 19, S37-S43, 2000; Oguz H, et al.: The height and radius of tear meniscus and methods for examining these parameters. Cornea 19, 497-500, 2000; Yokoi N, et al.: Tear meniscus changes during cotton thread and schirmer testing. Invest Ophthalomol Vis Sci 41, 3748-3753, 2000; Yokoi N.: meniscometry and video meniscometer, Atarashii Ganka 17, 65-66, 2000; Yokoi N., Nakamura Y.: New Examination of Sjögren syndrome, Atarashii Ganka 16, 1515-1523, 1999; Yokoi N: meniscometry, Medical care of dry eye PPP, 80-83, 2002, Dry Eye kenkyukai ed., MEDICAL VIEW CO., LTD., Tokyo, 2002; Japanese Patent Unexamined Publication No. H11-267102, etc). This measuring method permits an accurate and highly reproducible measurement. In addition to this method, observation of tear meniscus (Yokoi Y.: observation of tear meniscus, Medical care of dry eye PPP, 25-28, 2002, Dry eye Kenkyukai, MEDICAL VIEW CO., LTD., Tokyo, 2002) and methods based on the measurement of various parameters (height, cross-sectional area, depth, etc.) (Kunio Maruyama et al.: Evaluation of rapport of lacrimal duct using digital imaging in treatment of dry eyes, Nippon Ganka Gakkai Zasshi (Journal of Japanese Opthalmological Society) 107, 526-529, 2003; Mainstone JC, et al.: Tear meniscus measurement in the diagnosis of dry eye. Curr. Eye Res. 15, 653-661, 1996; Oguz H, et al.: The height and radius of the tear meniscus and methods for examining these parameters. Cornea 19, 497-500, 2000), an interferometry method (Norihiko Yokoi: interferometry of tear, Atarashii Ganka 14, 1337-1338, 1997; Yokoi N, et al.: Correlation of tear lipid layer interference patterns with the diagnosis and severity of dry eye. Am J Ophthalmol 122, 818-824, 1996; Norihiko Yokoi, You Nakamura: New Examination of Sjögren syndrome, Atarashii Ganka 16, 1515-1523, 1999) and a fluorophotometry method (Yokoi N. et al.: New fluorophotometer, Atarashii Ganka 11, 1043-1045, 1994; Yokoi N, Kinoshita S: Clinical evaluation of corneal epithelial barrier function with slit-lamp fluorophotometer. Cornea 14, 485-489, 1995; Norihiko Yokoi: Investigation of ophthalmology examination - fluorophotometry of anterior eye part, Clinical Ophthalmology 52, Special issue, 11, 76-77, 1998,), and the like, may be used.

Note here that in the above mention, each embodiment is described with reference to examination (diagnosis) of dry eyes as an example. However, diseases to which the lacrimation examination method of the present invention may be applied are not limited to dry eye. The present invention can be applied to various ocular surface diseases whose assessment is based on or related to static or dynamic parameters of lacrimation, and the change thereof, and the like.

The subjects (test subjects) of the lacrimation examination using the reagent for lacrimation examination of the present invention are typically mammalian animals. Specific examples thereof can include human, monkey, chimpanzee, dog, cat, horse, cow, and the like. Among them, a preferable subject is human.

Another aspect of the present invention relates to another application of the above-mentioned Thiopropanal S-Oxide and analogues thereof (represented by the following chemical formula). Specifically, another aspect of the present invention relates to a lacrimation promoter composition containing as an active ingredient a compound represented by the following chemical formula. Hereinafter, the corresponding descriptions above are applied to matters that will not be particularly specified below. 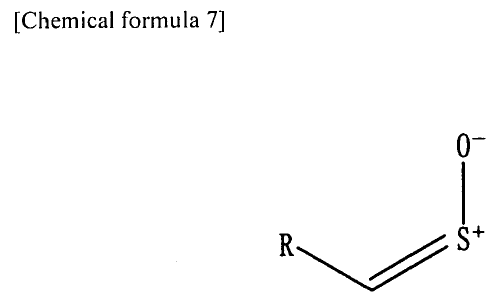 wherein R represents a C₁₋₅ alkyl chain.

The lacrimation promoter composition of the present invention can be used as a therapeutic drug for various diseases that are effectively prevented or treated by promoting the secretion of tears. Dry eye may be mentioned as a representative example of the diseases that are subject to the lacrimation promoter composition of the present invention. In addition, the lacrimation promoter composition of the present invention can be used as a therapeutic drug for preventing or treating diseases of the ocular surface, for example, a group of diseases that are accompanied by a corneal epithelium disorder. Furthermore, the lacrimation promoter composition of the present invention can be used for a prevention of dryness of the ocular surface accompanying VDT (Visual display terminal) work, for a postoperative protection of the ocular surface (for example, after corneal transplantation, after refractive operation, after cataract operation, and the like), for a relief of symptoms or alleviation of discomfort in ocular allergy, and the like.

The lacrimation promoter composition of the present invention can be prepared into any arbitrary forms such as an eye drop, ophthalmic ointment, and the like. However, since the active ingredient thereof is volatile, the lacrimation promoter composition is suitably used in a non-contact manner. The phrase "used in a non-contact manner" herein denotes exposing an eye or eyes to the vaporized active ingredient by placing the lacrimation promoter composition of the present invention in the vicinity of the eye(s) to be treated instead of directly dropping it or applying it to the eye. For example, by placing a container (exposure container) containing vaporized active ingredient over the eye with the opening thereof facing the eye, the above-mentioned "used in a non-contact manner" can be achieved. Note here that the lacrimation promoter composition of the present invention may be supplied to the user in a combination (i.e., a kit) with such an exposure container.

The lacrimation promoter composition of the present invention contains an active ingredient (Thiopropanal S-Oxide and/or the analogous compounds thereof) in an amount that is necessary for obtaining an expected treatment (or prevention) effect. The amount of the active ingredient in the lacrimation promoter composition is adjusted to a range so as to deliver the desired dose although it depends on diseases to be treated, application mode, dosage forms, and the like. For example, it is preferable that the amount of the active ingredient of the lacrimation promoter composition can be adjusted to the amount that induces secretion of about 25 µl to about 30 µl of tears. It is preferable that the concentration of the present promoter composition is adjusted so that the above-mentioned amount of tears is secreted by a single dose.
In order to achieve the above mentioned effect, for example, an amount of Thiopropanal S-oxide can be added in the lacrimation promoter composition of the present invention so that the amount of Thiopropanal S-oxide contained in 3 µl of ethyl ether solution of an area value of about 1,700, 000 can be applied in a single dose.
The amount of tears secreted by a single dose of the lacrimation promoter composition of the present invention, on condition that the dose will be repeated, is an amount of tears capable of appropriately effecting water supply and wash out effect, and the like, to the eyes of patients of a disease such as dry eye. When conventional artificial tears are dropped in the above-mentioned tear amount several times, the physiological composition of the natural tears is significantly altered, and the stability of the tear is compromised. There is also a problem associated with the effect of the preservatives used in the artificial tears (ideal artificial tears containing all the components contained in natural tears have not been developed yet). In contrast, if the lacrimation promoter composition of the present invention is used, since water supply can be accomplished by the tears of the patients themselves, an epoch-making treatment effect for dry eye, and the like, can be achieved. The maximum effect is obtained from a non-contact type lacrimation promoter composition.

The lacrimation promoter composition of the present invention can be prepared with appropriate isotonizing agents, buffers, surfactants, stabilizing agents, chelating agents, preservatives, and the like, in addition to the above-mentioned active ingredient. Examples of the isotonizing agents include sodium chloride, glycerin, and the like. Examples of the buffers include a borate buffer, a phosphate buffer, an acetate buffer, a citrate buffer, a Tris buffer, and the like. Examples of the surfactants include polyoxyl 40 stearate, polyoxyethylene hydrogenated castor oil, and the like. Examples of the stabilizing agents include sugars (glucose, mannose, galactose, fructose, mannitol, inositol, xylitol, maltose, lactose, dextran, chondroitin sulfuric acid, hyaluronic acid, and the like), cellulose derivatives (methyl cellulose, ethyl cellulose, hydroxyethyl cellulose, hydroxypropyl cellulose, hydroxypropyl methyl cellulose), sodium citrate, sodium edetate, and the like. Examples of the chelating agents include sodium edetate, citric acid, and the like. Examples of the preservatives include benzalkonium chloride, paraben, chlorbutanol, thimerosal, hydrogen peroxide, and the like.

It is preferable that when the lacrimation promoter composition of the present invention is used in a form such as an eye drop, etc., which is applied to the eyes directly, the pH is adjusted to about pH 5 to about pH 8.

It is preferable that the dosage amount of the lacrimation promoter composition of the present invention is set so that an expected effect can be sufficiently obtained and that no or therapeutically negligible side effects will occur. When setting the dosage amount, the type of the disease, the severity of the disease, age and the like, of a patient to whom the lacrimation promoter composition is applied may be taken into consideration. Note here that any person skilled in the art should be able to set an appropriate dosage amount by considering these matters. The dosage frequency may be selected from 1 to 3, 4 to 6, or 7 to 10 times a day and so forth. When the lacrimation promoter composition of the present invention is prepared as an eye drop, the amount of use for each application may be 1 to 6 drops, preferably 1 to 4 drops, and more preferably 1 to 3 drops. When planning a dosage schedule, such factors as conditions of the patient, duration of the lacrimatory effect, and the like, may be taken into consideration.
In the present invention, the concentration of the lacrimation promoter composition and the dosage amount can be determined based on the results obtained by the above-mentioned lacrimation examination methods, and in accordance with the level of symptoms or the severity of the diseases, and the like. From the tear volume measured in each patient, and the like, the amount of the active ingredient of the lacrimation promoter composition may be determined for each patient, and by using the most appropriate prescription for the patient, , therapeutic effect on dry eyes, and the like, can be obtained by utilizing the natural tears of the patient him- or herself.

### [Example 1]

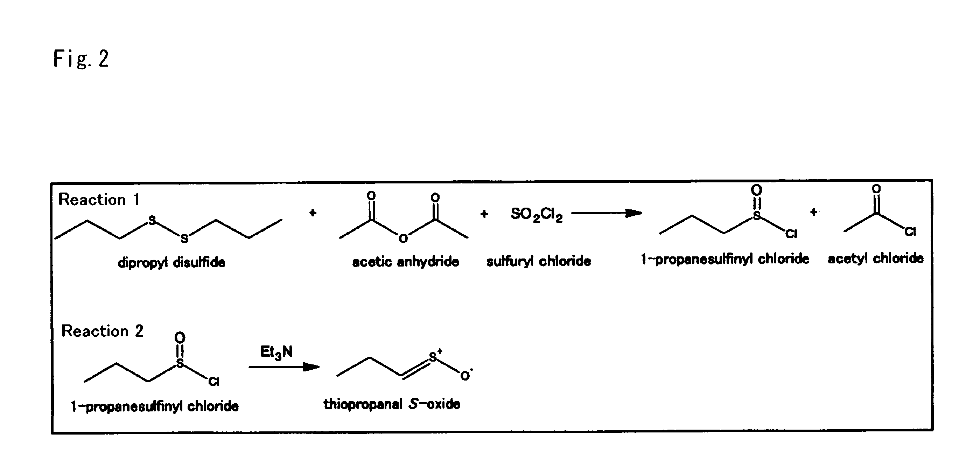

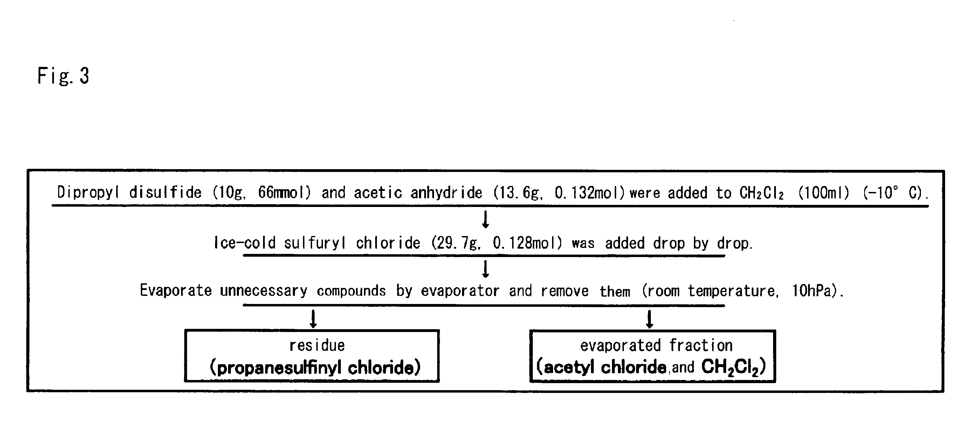

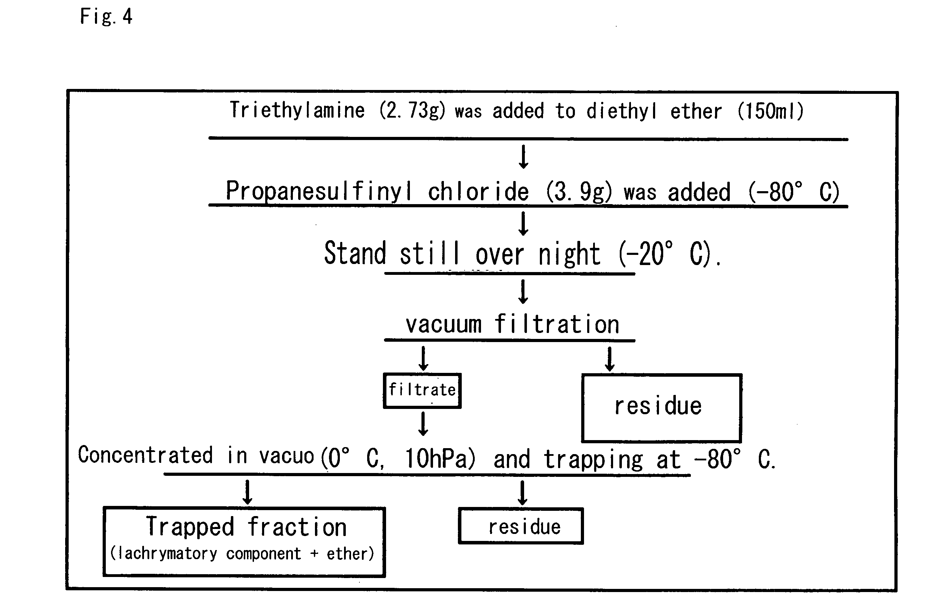

1. Preparation of lacrimatory component
The lacrimatory component (Thiopropanal S-oxide) used in the following Examples was chemically synthesized in accordance with the method described in J. Am. Chem. Soc. Vol. 118, No. 32, 1996, 7492-7501. Fig. 2 shows a synthesis flow. This method of synthesis includes two steps of reactions: a step of synthesizing 1-propanesulfinyl chloride (reaction 1) and a step of synthesizing the lacrimatory component (Thiopropanal S-oxide) from 1-propanesulfinyl chloride (reaction 2). Specific synthesis procedures and the experimental results are described below.
1-1. Reaction 1
Fig. 3 shows a synthesis scheme of 1-propanesulfinyl chloride. After evaporation of the solvent, 17.12 g of residues were obtained. The structure of thus obtained 1-propanesulfinyl chloride was confirmed by ¹H-NMR.
1-2. Reaction 2
Fig. 4 shows a synthesis scheme of lacrimatory component (Thiopropanal S-oxide) from the 1-propanesulfinyl chloride. When the lacrimatory component was synthesized according to the scheme shown in Fig. 4, the ethyl ether solution containing the resulting lacrimatory component maintained the lacrimatory property excellently. Finally, the ethyl ether solution was concentrated about 5 times by evaporating ethyl ether at 0°C and under 220 hPa, to obtain an ethyl ether solution containing a lacrimatory component to be used for the subsequent experiments (hereinafter, referred to as "lacrimatory component solution"). The structure of the synthesized lacrimatory component was confirmed by ¹H-NMR.
1-3. Measurement of the amount of lacrimatory component in the lacrimatory component solution
The lacrimatory component solution (1 µl) was subjected to HPLC and the peak area of the lacrimatory component obtained (hereinafter, referred to as an "area value") was measured. This area value was used as a provisional measure of the amount of the lacrimatory component in 1 µl of the lacrimatory component solution. The mean of the area value measured for the lacrimatory component solution used in the Examples was 1704876 (n=3). The conditions of HPLC were as follows:

| (HPLC conditions) | |
|---|---|
| Instrument used: | SHIMADZU 10A Series |
| Ultraviolet and visible light detector: | SHIMADZU SPD-10AV |
| Chromatographic pack: | SHIMADZU CR-5A |
| Column: | Pegasil ODS (Senshu Scientific co., ltd) |
| Solvent: | 30% MeOH (pH 3.3, TFA) |
| Flow rate: | 0.6 ml/min |
| Column temperature: | 35°C |
| Detection wavelength: | 210 nm |

### [Example 2]

By using the lacrimatory component solution prepared in Example 1, the lacrimatory effect of the lacrimatory component (Thiopropanal S-oxide) was investigated.
1. Subjects (test subjects)
   The subjects included six normal male subjects (mean age: 21.7) regularly wearing soft contact lenses (SCL).

2. Items examined
   1) Lacrimation effect of a lacrimatory component (Thiopropanal S-oxide) (presence or absence of a lacrimation effect, duration of the effect (duration time, transient nature of the effect), the effect of the lacrimation on the contralateral non-exposed eye)
   2) Influence of a solvent (ethyl ether) on the lacrimation
   3) Influence of nasal exposure on the lacrimation
   4) Influence of an SCL wear on the lacrimation
   5) Influence of topical application of an eye-drop anesthesia on the lacrimation

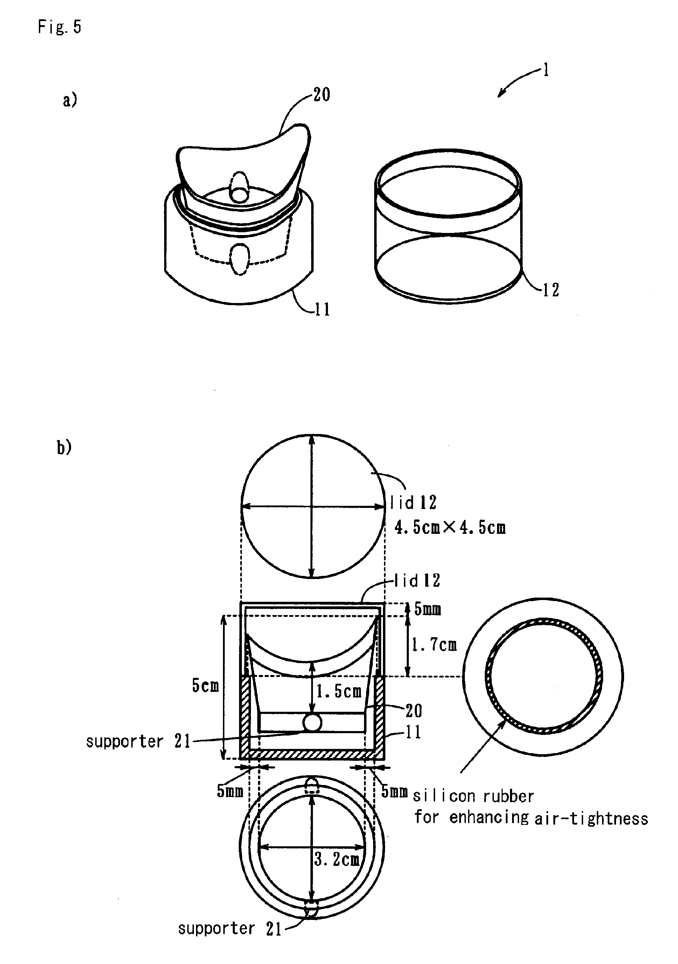

3. Methods of examinations and evaluations
   3-1. Exposure method
      Fig. 5 shows an exposure container. Note here that Fig. 5a is a perspective view of the exposure container 1 with its closure lid removed. Furthermore, Fig. 5b shows a detailed configuration of the exposure container 1 with its lid on. The exposure container 1 comprises an outer container consisting of an outer container main body 11 and a lid for outer container 12 and an inner container 20. The outer container is of a cylindrical shape, and when the lid is on, has a height of about 5 cm and a diameter of about 4.5 cm. The shape of the lid 12 for the outer container is not particularly limited, if it can be attached and removed to and from the outer container main body 11 so that it covers the upper portion of the inner container completely when it is closed and the opening (mouth) of the inner container 20 is exposed when the lid is removed. When the lid is closed, the inside of the outer container can be hermetically sealed. The inner container 20 has the same shape as a commercially available eye washing cup. Specifically, the inner container 20 has a substantially cup shape with its upper part open and the cross sectional area of the inner space gradually tapering off toward the bottom. The upper circumference of the opening of the inner container 20 has concave curvature (as shown in Fig. 5) so that the mouth of the cup fits snugly on the periphery of the subject's eye. The plane view of the upper part of the inner container 20 is circular with a diameter of about 4.2 cm. The plane view of the bottom of the inner container 20 is circular with a diameter of about 3.2 cm. The inner container 20 is fixed in the outer container main body 11 via a supporter part 21. All of the outer container main body 11, the lid 12 of outer container and the inner container 20 are made of acrylic. Note here that the upper ridge of the outer container main body 11 is fitted with a silicone rubber ring in order to effect the air-tight seal when the lid is closed.
      In the above-mentioned exposure container 1, since the opening of the inner container 20 is designed to have the same shape as that of an eye washing cup, only by holding the outer container with a single hand, the upper part of the inner container 20 can be fitted easily on the periphery of the eye of the subject being examined.
      Note here that the above-mentioned exposure container 1 can be used for any of the exposure containers included in lacrimation examination kits or lacrimation promoter kits.
      By using the above-mentioned exposure container, exposure is accomplished by the following procedures.
      (1) Firstly, a circular piece of filter paper having a diameter of 3 mm is instilled with the lacrimatory component solution prepared by the method described in Example 1 by applying 3 µl of the solution with a micropipette. Thereafter, the filter paper is transferred to the inside of the inner container of the exposure container and then the lid of the outer container is closed. After 15 seconds, the lid of the outer container is removed and closed again immediately, and after 30 seconds, the same procedure is repeated once again to remove ether vapor from the inner cavity of the inner container(ether vaporization operation).
      (2) After the above-mentioned operation, the lid of the outer container is removed again and the upper part of the inner container of the exposure container is tightly fitted onto the periphery of the eye, so that the ocular surface is exposed to the vapor of the lacrimatory component (exposure operation).
      Note here that when exposing an eye to the solvent (ethyl ether) only solution (examination item 2) the same procedure as described above is to be followed except that ethyl ether concentrated to 5-times concentration is used in place of the lacrimatory component solution. Furthermore, when nasal exposure is to be carried out (item 3 of the list of items examined), after the ether vaporization operation, the lid of the outer container of the exposure container is removed and then the upper part of the inner container of the exposure container is placed in the vicinity of a nostril to expose subject's nasal passage to the lacrimatory component solution. At this time, the non-exposed nostril is closed with a finger tip.

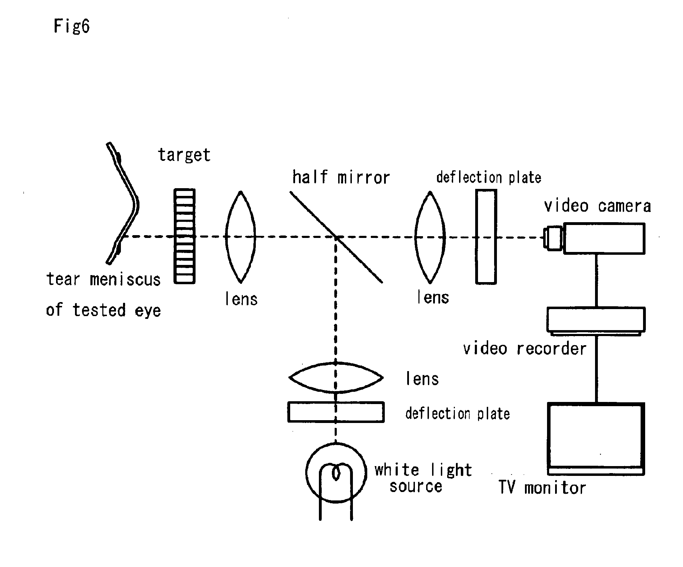

3-2. Evaluation of tear volume increase
   The increase of the radius of tear meniscus curvature (mm): "ΔR (t minutes) = R (t minutes after exposure, t = 2, 5, 1 0)-R (before exposure)" is measured by video meniscometry (see, for example, Yokoi N, et al.: Reflective meniscometry: a non-invasive method to measure tear meniscus curvature. Br J Ophthalmol, 83, 92-97, 1999; Yokoi N, et al.: Reflective meniscometry: a new field of dry eye assessment. Cornea 19, S37-S43, 2000; Oguz H, et al.: The height and radius of the tear meniscus and methods for examining these parameters. Cornea 19, 497-500, 2000; Yokoi N, et al.: Tear meniscus changes during cotton thread and Schirmer testing. Invest Ophthalomol Vis Sci 41, 3748-3753, 2000; Yokoi N.: meniscometry and video meniscometer, Atarashii Ganka 17, 65-66, 2000; Yokoi N, You Nakamura: New Examination of Sjögren syndrome, Atarashii Ganka 16, 1515-1523, 1999; Yokoi N: meniscometry , Medical care of dry eye PPP, 80-83, 2002, Dry Eye kenkyukai ed., MEDICAL VIEW CO., LTD., Tokyo, 2002; Japanese Patent Unexamined Publication No. H11-267102, etc.). Fig. 6 shows a basic configuration of the video meniscometer used for the measurements. Since the tear meniscus is optically equivalent to a concave mirror, when a target image is projected by the video meniscometer, a small reflected image is formed in front of the meniscus. In the video miniscometer, a light-transmitting system and a light-receiving system are positioned coaxially and they can be rotated freely in the range of 180° in a horizontal plane. Furthermore, since the target can be rotated in the range of 360°, any tear meniscuses formed on the ocular surface can be recorded on the video while approaching in front of the subject. By recording a target image on a digital video while monitoring the target image on a display, a real time image can be printed out clearly. Examination using the video meniscometer can be carried out by the following procedure. In order not to induce reflex lacrimation, the irradiant light is moved upward from the lower eyelid to project the target onto the tear meniscus. Since the lid margin is convex with respect to the horizontal front direction and the position of the top is different in individual subjects, a light-transmitting system is rotated in the horizontal plane until the image on the monitor appears horizontal. The test subject is instructed to avoid yawning or gazing, which may promote lacrimation, and to be in a natural condition. The concave portion of the tear meniscus is located in a somewhat upward position from the lid margin. In the measurement (meniscometry) of the actual radius of tear meniscus curvature, the stripe width of the lattice of the printed image is measured by using a microruler, and the radius is calculated using the concave mirror formula. Alternatively, video image is captured and the radius of curvature is measured by using image analysis software on a personal computer.

3-3. Evaluation of degree of stimulation
   Degree of stimulation was evaluated using both a VAS (Visual analog scale; test subjects are asked to indicate the degree of stimulation felt by marking on a 10 cm long line scale (0 cm:= no stimulation and 10 cm = intolerably strong stimulation), and a four-level category rating (0: no stimulation, 1: slight stimulation, 2: tolerable stimulation, 3: intolerably strong stimulation).

The examination method for each item examined was as follows.
3-4. Effect of lacrimatory component on naked eye: examination item 1
   (1) For each subject, the eye to be exposed to the lacrimatory component (exposed eye) is determined using an envelope method.
   (2) The tear meniscus of both eyes on the baseline (before exposure) are observed and recorded.
   (3) The ocular surface is exposed to the lacrimatory component by the above-mentioned method.
   (4) Time taken for the stimulation to be felt (ST) and the longest exposure time are measured. Furthermore, after the exposure, at each minute up to 10 minutes, the change of the tear meniscus image (the increase in the radius of tear meniscus curvature) of the exposed eye and the contralateral non-exposed eye is observed and recorded by video meniscometry. The degree of stimulation is assessed using VAS when the stimulation begins to be felt, when the maximum stimulation is felt, and when examination is completed.

3-5. Influence of solvent on lacrimation: examination item 2
   (1) The eye to be exposed is determined using an envelope method.
   (2) The tear menisci of both eyes before exposure are observed and recorded.
   (3) The ocular surface is exposed to 5-times concentrated ethyl ether by the above-mentioned method. Note here that exposure time is 10 seconds.
   (4) The change of the image of the tear meniscus (the increase in the radius of tear meniscus curvature) right after exposure is observed and recorded by video meniscometry. At the same time, the degree of stimulation is evaluated by VAS. Furthermore, conditions of the cornea and conjunctiva after exposure is examined by slit microscopy.

3-6. Influence of the nasal exposure on the lacrimation: examination item 3
   (1) For each subject, the nostril to be exposed to the lacrimatory component is determined by an envelope method.
   (2) The subject was asked to wear goggles.
   (3) The nostril is exposed to lacrimatory component by the above-mentioned method with the other nostril closed with a finger tip.
   (4) The time taken for the stimulation to be felt (ST) and the changes of the images of the tear meniscus (increases of the radii of tear meniscus curvature) for both eyes are observed and recorded.

3-7. Effect of SCL wear on lacrimation: examination item 4
   (1) For each subject, the eye to be exposed is decided by an envelope method.
   (2) The subject is allowed to wear an SCL in the eye to be exposed and to become acclimated to it for 15 minutes.
   (3) The tear meniscus of both eyes before exposure are observed and recorded.
   (4) The ocular surface is exposed to the lacrimatory component by the above-mentioned method.
   (5) Time taken for the stimulation to be felt (ST) and the longest exposure time tolerated by the subject are measured. Furthermore, after the exposure, at each minute up to 10 minutes, the change of the tear meniscus image (the increase in the radius of tear meniscus curvature) of the exposed eye and the contralateral non-exposed eye is observed and recorded by video meniscometry. The degree of stimulation is evaluated using VAS when the stimulation is first felt, when the maximum stimulation is felt, and when the examination is completed.

3-8. Effect of eye-drop anesthesia on lacrimation: examination item 5
   (1) For each subject, the eye to be exposed is determined by an envelope method.
   (2) Two drops of Benoxil are instilled onto the eye to be exposed. The increased tear is removed by absorbing it with a micro-sponge one minute later so that the image of the tear meniscus returns to the base line level.
   (3) The ocular surface is exposed to the lacrimatory component by the above-mentioned method.
   (4) Time taken for the stimulation to be felt (ST) and the longest exposure time are measured. Note here that the exposure is continued until the stimulation is no longer tolerated or for 60 seconds at longest. Furthermore, after the exposure, at each minute up to 10 minutes, the change of the image of the tear meniscus (the increase in the radius of tear meniscus curvature) of the exposed eye and the contralateral non-exposed eye is observed and recorded by video meniscometry. The degree of stimulation is assessed using VAS when the stimulation is first felt, when maximum degree of stimulation is felt, and when the examination is completed. Note here that the same examinations are also performed using 4% xylocaine as the anesthetic.

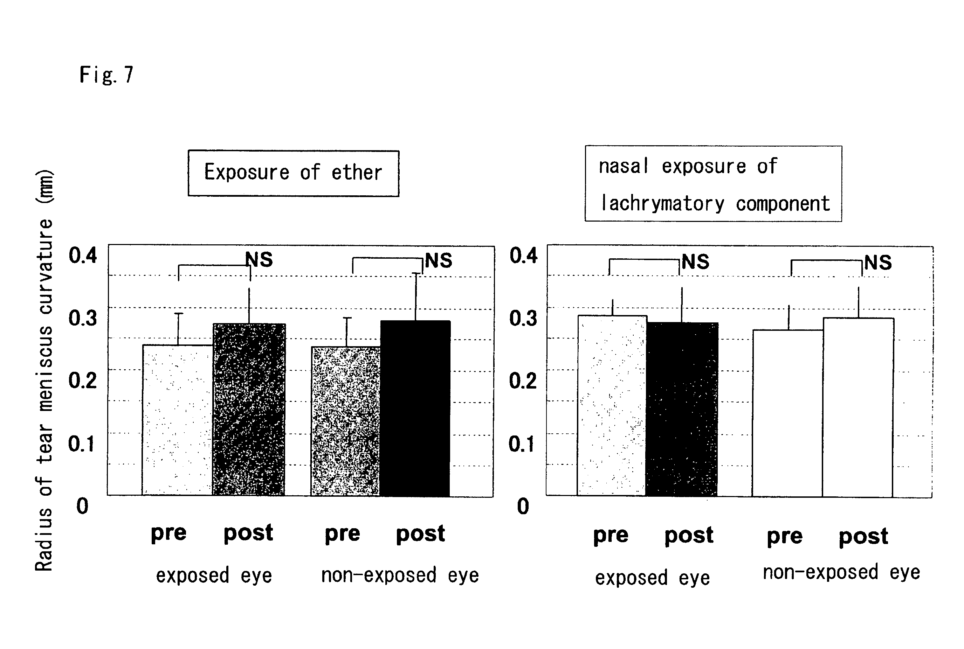

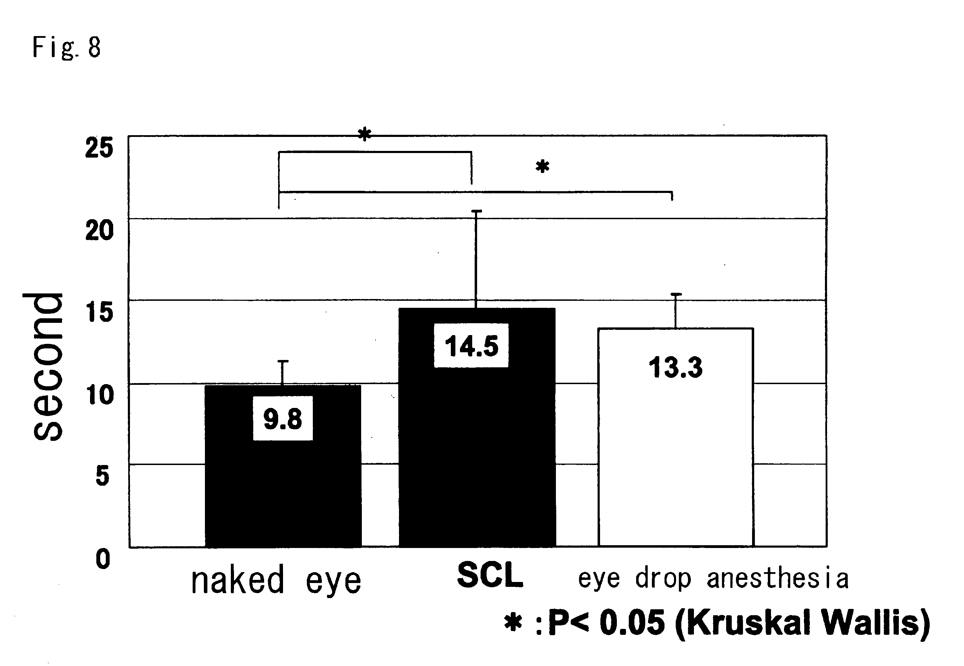

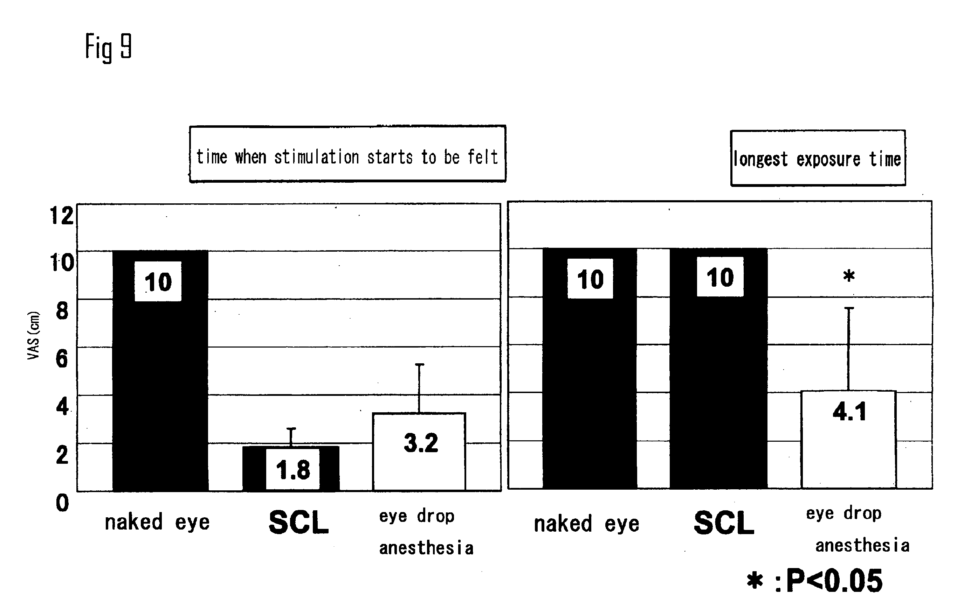

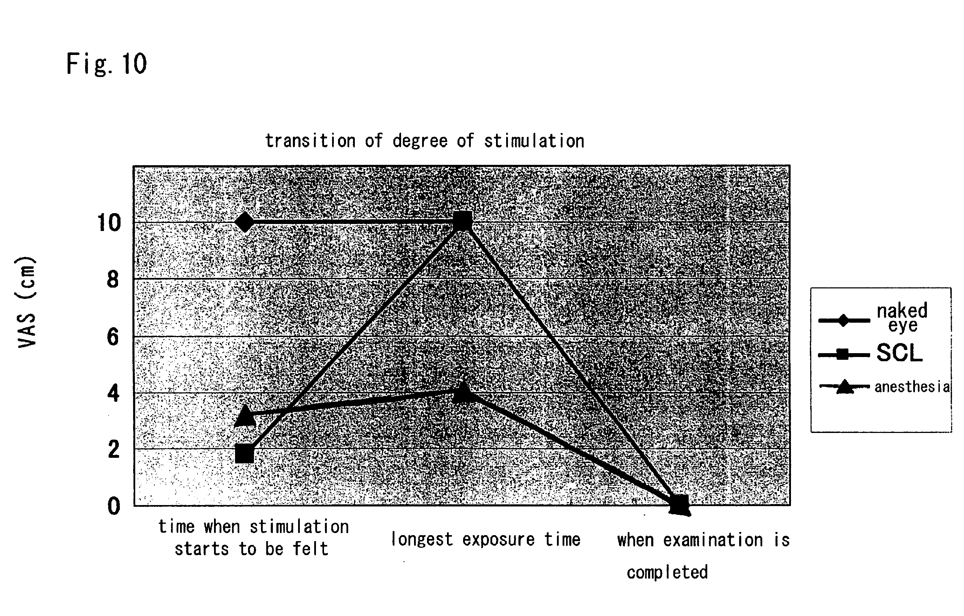

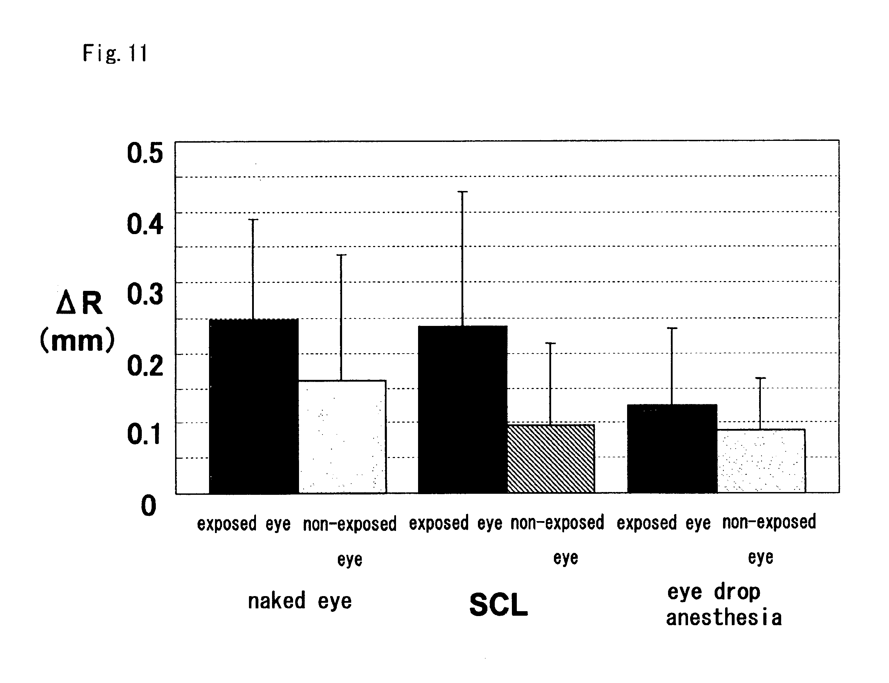

4. Results
   1) The experimental results on the influence of ether is shown in the left graph of Fig. 7. This graph shows that no reflex lacrimation is induced due to ethyl ether used as a solvent of the lacrimatory component.
   2) The experimental results on the influence of nasal exposure is shown in the right graph of Fig. 7. This graph shows that no reflex lacrimation is induced by the nasal exposure of the lacrimatory component.
   3) The measurement results of the time taken for the stimulation to be felt (ST) is shown in Fig. 8. In Fig. 8, STs measured in naked eyes, in eyes with SCL, and in eyes under eye-drop anesthesia are compared. The mean STs were 9.8, 14.5, and 13.3 (all values are in second) for naked eyes, eyes wearing SCL, and eyes under eye-drop anesthesia, respectively. The result shows that ST is longer in eyes with SCL and eyes under eye-drop anesthesia (p<0.05).
   (4) Based on four-level category rating, the mean degree of the stimulation at the longest exposure time was 3 in naked eyes, 3 in eyes with SCL, and 1.5 in eyes under eye-drop anesthesia (p<0.05). The degree of stimulation became 0 at the end of examination. Furthermore, the evaluation by VAS yielded results similar to those in the four-level category rating (Figs. 9 and 10). Note here that the left graph of Fig. 9 compares the degree of stimulation measured at the time when the stimulation starts to be felt in naked eyes, eyes wearing SCL, and eyes under eye-drop anesthesia. On the other hand, the right graph of Fig. 9 compares the degree of stimulation in naked eyes, eyes wearing SCL, and eyes under eye-drop anesthesia measured at the longest exposure time. Fig. 10 shows a comparison of the change in the degree of stimulation over time measured by VAS.
   5) The mean increase in the radius of tear meniscus, ΔR (mm), measured at 2, 5, and 10 minutes after exposure were, respectively, 0.25 (in three eyes which could be exposed for longer than 20 seconds, tears overflowed from the lid margin and the overflowed amount was not reflected in the ΔR for those eyes), 0.013 and 0.0047 in naked eyes; 0.24, 0.0092, and -0.0017 in eyes with SCL; and 0.15, 0.033, and 0.0098 in eyes under eye-drop anesthesia. The ΔR decreased over time. In three subjects whose naked eye was exposed for longer than 20 seconds, the ΔR increased in the contralateral non-exposed eye as well (0.30 mm). A comparison of the tear volume increases two minutes after the exposure is shown in Fig. 11.

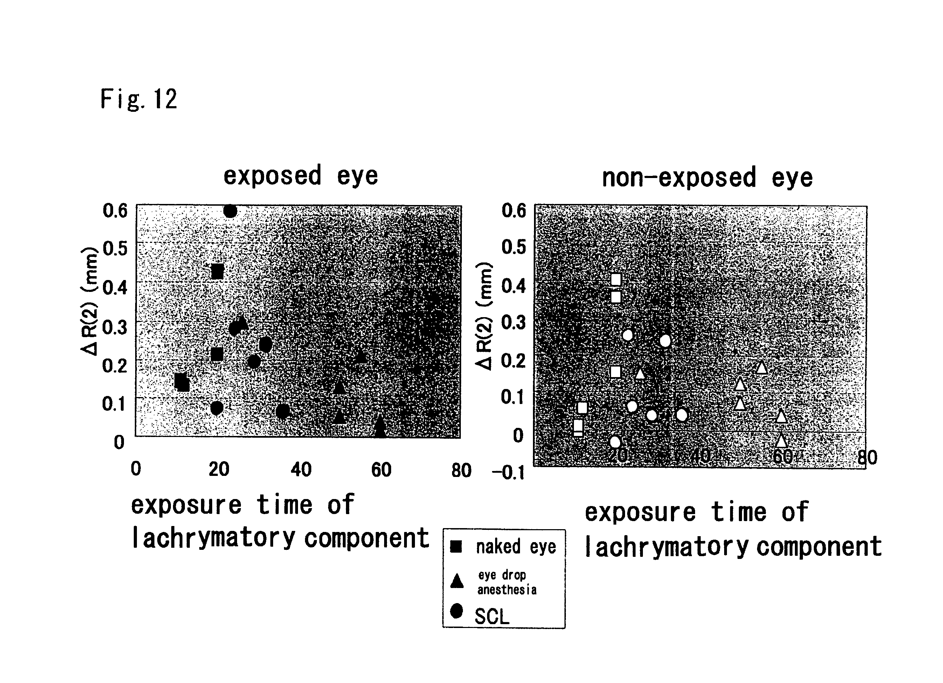

   6) Fig. 12 shows a graph summarizing the relationship between the exposure time and the tear volume increase. It is shown that, when exposure is carried out for longer than 20 seconds, lacrimation is induced in the contralateral non-exposed eye.
The above-mentioned results can be summarized as follows: (1) stimulation by a lacrimatory component is transient; (2) the lacrimation inducing effect by the stimulation is strong enough to induce lacrimation in the contralateral non-exposed eye; and (3) any adverse or lingering effects of the exposure of a lacrimatory component on the ocular surface is not found; and (4) the induction of lacrimation by a lacrimatory component is presumably related to corneal perception. It is clarified from this Example that a lacrimatory component (Thiopropanal S-oxide) can satisfy conditions, for example, exhibiting the high lacrimation effect, which are required as a reagent for lacrimation examination. That is to say, it is revealed that the lacrimatory component (Thiopropanal S-oxide) is effective as a reagent for lacrimation examination. Furthermore, since the lacrimatory component (Thiopropanal S-oxide) has a high lacrimation inducing effect, it is effective not only as a reagent for lacrimation examination but also as a preventive or therapeutic drug for dry eye. Note here that the fact that the stimulation is transient and the persisting influence on the ocular surface is not substantially seen means that the component imposes extremely low burdens on test subjects or patients. This is an advantage of the lacrimatory component (Thiopropanal S-oxide) in clinical application thereof.

### [Example 3]

The effect of the lacrimatory component (Thiopropanal S-oxide) on the lacrimation and the dynamic change thereof in tear deficient dry eye patients were examined.
1. Subjects (test subjects)
   The subjects included 16 patients with tear deficient dry eye [15 female and I male, mean age=62.4], who were subdivided into a group of 10 slight to moderate patients receiving eye-drop treatment (slight to moderate patient group): 10 eyes, and a group of 6 patients with severe dry eye receiving upper and lower punctual plug occlusion (severe patient group): 6 eyes], and a control group of 6 normal subjects [6 eyes] (mean age= 21.7).

2. Examination and Evaluation method
   (1) For each subject. the eye to be exposed is determined. In the slight / moderate dry eye patient group, the eye with severer corneal epithelium disorder determined by the result of fluorescein staining examination under the slit-lamp microscope is selected as an exposed eye. In the severe patient group, the eye with upper and lower punctual plug occlusion is selected as the eye to be exposed.
   (2) The ocular surface is exposed to the lacrimatory component. Note here that the exposure is continued until the stimulation is no longer tolerated or for 60 seconds at longest.
   (3) Time taken for the stimulation to be felt (ST) and the longest exposure time for individual patients are measured. In addition, at every minute up to 10 minutes after the exposure, the change of the image of the tear meniscus (the increase in the radius of tear meniscus curvature) is observed and recorded by video meniscometry and compared with that of normal subjects. Further, the degree of stimulation (VAS) is recorded when the stimulation starts to be felt, when the maximum stimulation is felt, and the examination was completed.
   (4) Finally, corneal epithelium is examined under slit-lamp microscopy for any signs of deterioration.
The measurement of the time taken for the stimulation to be felt (ST), the measurement of the longest endured exposure time, the observation and recording of the change of the image (the increase in reflex lacrimation) of meniscus, and the evaluation of the degree of stimulation (VAS) are carried out by the same methods as described in Example 2.

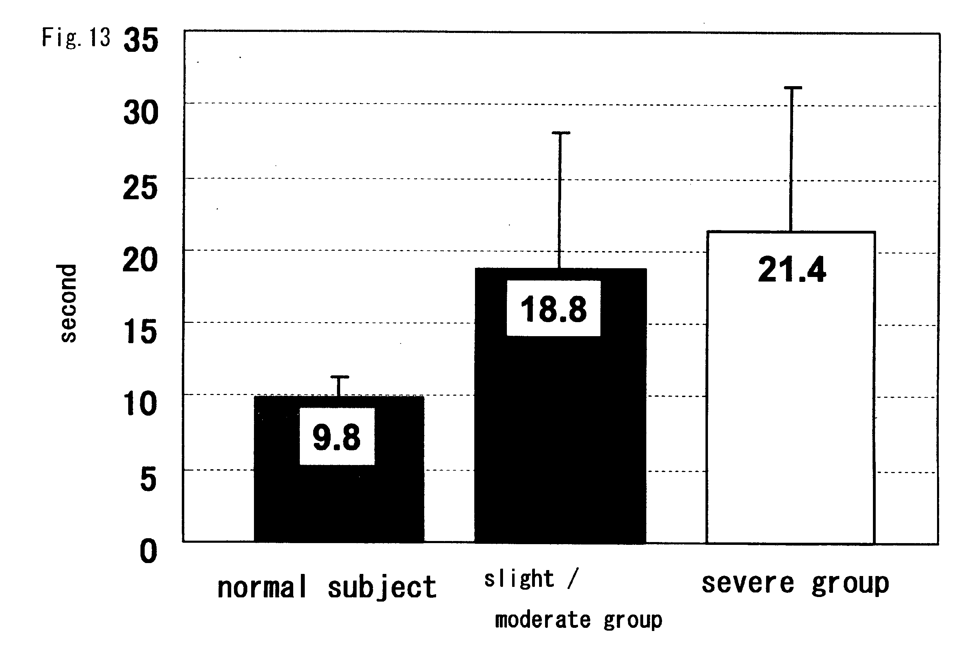

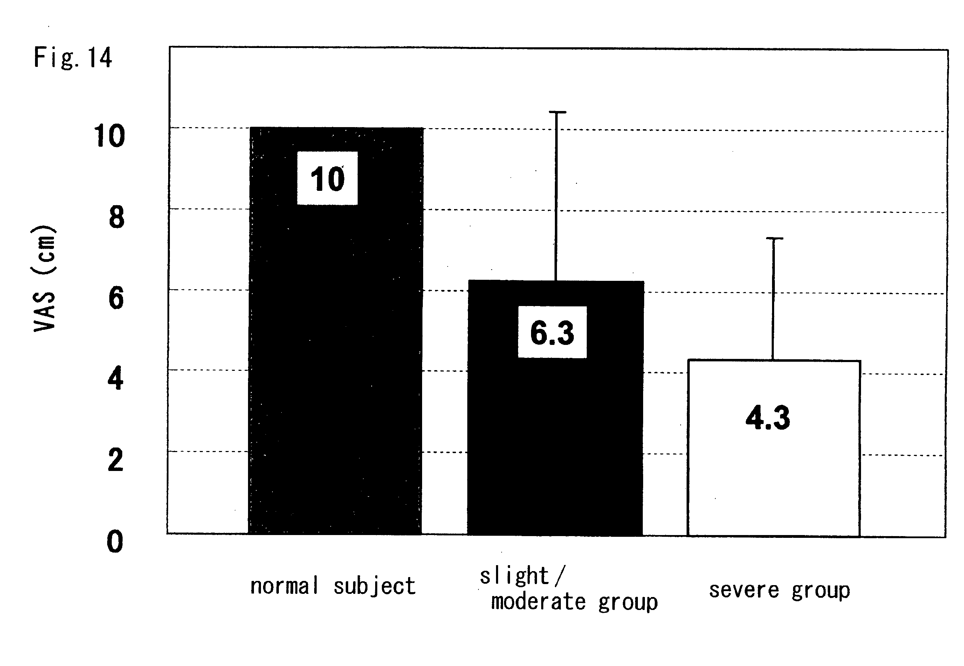

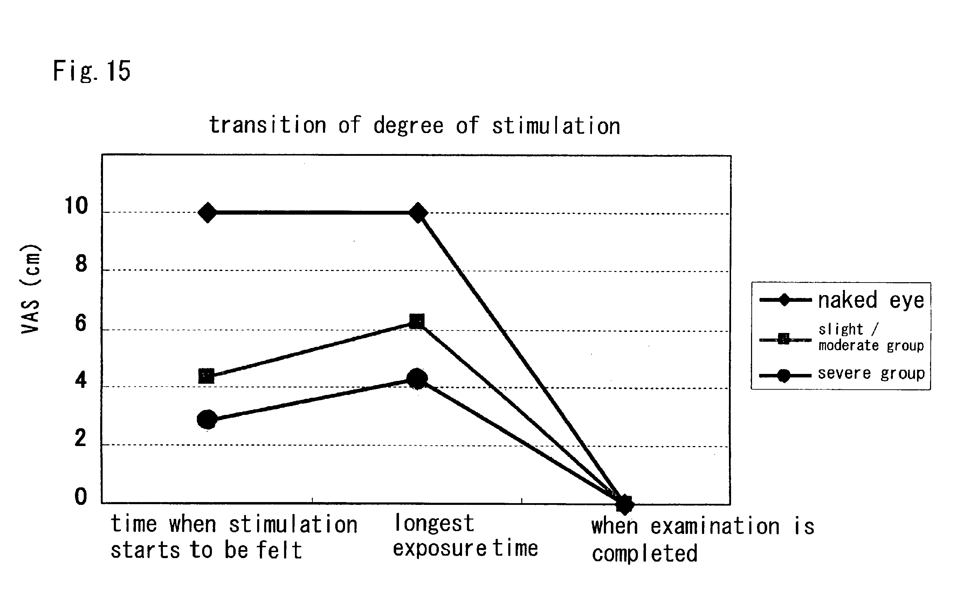

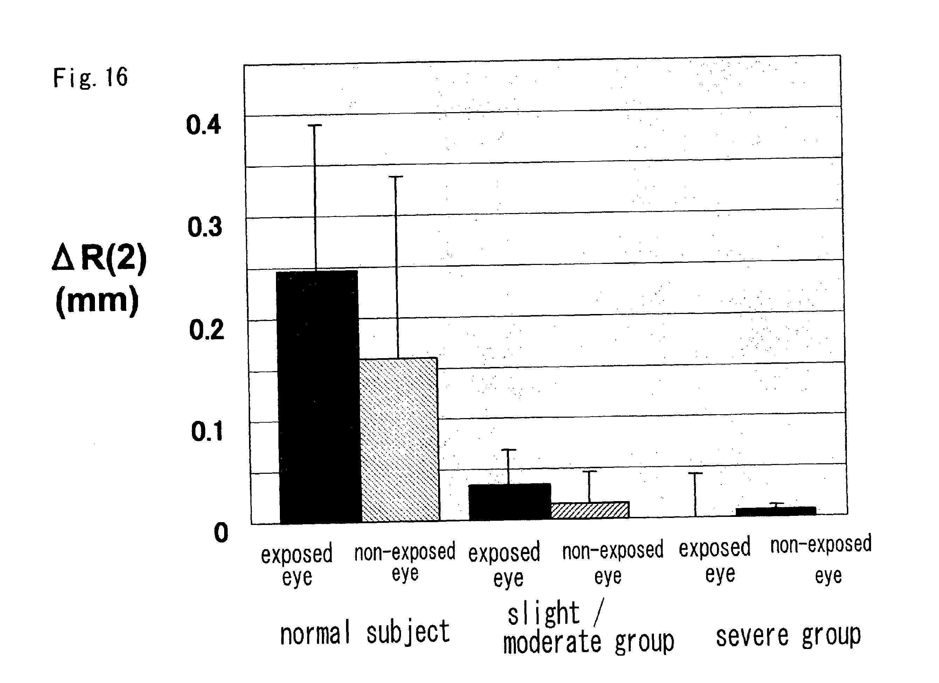

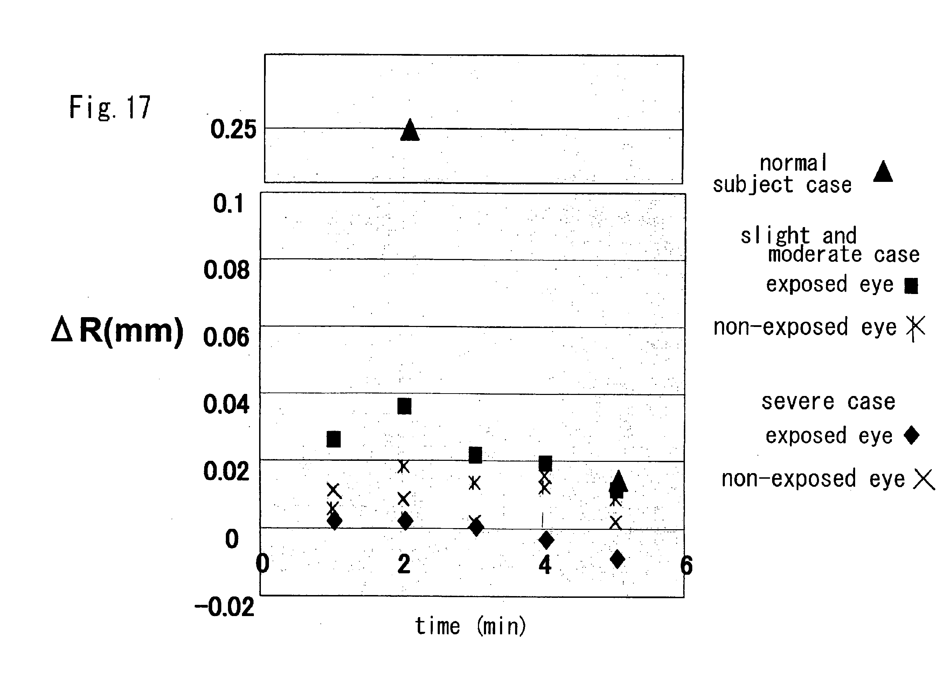

3. Results
   1) The measurement results of the time taken for stimulation to be felt (ST) are shown in Fig. 13. In the graph shown in Fig. 13, comparison of ST for normal subjects, the slight / moderate patient group and the severe patient group are made. ST values of the slight / moderate patient group and the severe patient group are 18.8±9.3 and 21.4±9.8 (mean value ± standard deviation; second), respectively. These ST values are significantly longer than the ST values for normal subjects (9.8±1.5) (in any comparison, p<0.05).
   (2) The mean degree of the stimulation based on four-level category rating is 1.8 in the slight / moderate patient group and 1.7 in the severe patient group at the longest exposure time. These values were lower than the mean value of 3.0 for normal subjects. In all cases, the values became zero when the examination was completed. No effects on the ocular surface epithelium was observed after completion of the examination. In addition, similar results to those obtained using the four-level category rating were obtained from the evaluations using VAS as well, (Figs. 14 and 15). Note here that Fig. 14 is a graph showing the comparison of results in VAS of the normal subjects, the slight / moderate patient group, and the severe patient group. On the other hand, Fig. 15 shows the transition of the results in VAS over time.
   3) The ΔR was maximum atΔR (2 minutes))[slight/moderate patient group: 0.034±0.03, severe patient group: 0.00042±0.04 for (these were both significantly lower (p<0.05) than that of the normal subjects (0.25±0.14) and substantially 0 at ΔR (5 minutes) [in normal subjects, the value reached the maximum by ΔR (1 minute), and returned to substantially 0 at ΔR (10 minutes)]. However, in three of six eyes of normal subjects, tear overflowed from the eye lid margin and the amount overflowed was not reflected in the ΔR. Note here that a comparison of the increase in the tear volume two minutes after the exposure is shown in Fig. 16 and the comparison of the change over time of the increase in the tear volume is shown in Fig. 17.
The above-mentioned results are summarized as follows. It is confirmed that: (1) the amount of reflex lacrimation induced by the exposure of a lacrimatory component is reduced in a tear deficient dry eye cases (severe case < slight and moderate cases < normal subjects); (2) tear deficient dry eye patients tend to feel the stimulation of the lacrimatory component less intense than normal subjects do, and the time taken for the stimulation to be felt becomes longer; and (3) the lacrimatory component can be applied in the medical field (in particular, examination of dry eye, or, in particular, application to prevention or treatment of slight to moderate cases of tear deficient dry eye and excessive tear evaporation type dry eye). Note here that the fact that the use of the lacrimatory component (Thiopropanal S-oxide) does not influence on the ocular surface epithelium means that the component imposes little burdens on test subjects or patients. This is an advantage of the lacrimatory component (Thiopropanal S-oxide) in the clinical application thereof,

### [Example 4]

The relationship between the extension of a tear lipid layer due to blinking and the tear volume was examined. Unless otherwise specified, the exposing method of the lacrimatory component and the measurement method of the tear volume were the same as those in the above-mentioned Examples.

1. Purpose
   The tear lipid layer on the cornea extends and contracts on a aqueous layer with blinking. However, in eyes highly deficient in tears, the lipid layer does not extend well on the cornea, and the lipid layer image often cannot be observed (Yokoi N, et al, AJO, 1996). That is to say, the extension of the tear lipid layer with blinking may be largely related to the tear volume. Therefore, the relationship between the tear volume on the ocular surface and the extension of the lipid layer (the relationship at some predetermined time during observation: examination item a) was examined. Furthermore, by inducing reflex lacrimation, the influence of tear volume increase on the extensibility of the lipid layer was examined (examination item b).

2-1. Subjects examined for item a
   The subjects included in total 82 cases, 123 eyes (4 male subjects, 78 female subjects, age= 58.4±12.4 (27 to 80)). More specifically, the subjects included 11 cases 19 normal eyes, and 71 cases, 104 tear deficient dry eyes (excluding eyes with Meibomian gland dysfunction).
2-2. Subjects examined for item b
   In total, the subjects included 35 cases 35 eyes (1 male subject, 34 female subjects, age= 61.3±12.9 (27 to 80)). All cases are tear deficient dry eyes.

3. Method
   The extensibility of the tear lipid layer was evaluated using DR-1 (registered trademark, Kowa Company ltd.). That is to say, the extensibility of the tear lipid layer on the cornea after blinking was classified based on the following criteria and evaluated: Grade (G) 1 (tear lipid layer extends smoothly on the entire surface); G2 (tear lipid layers extends slowly over more than 1/2 of the surface); G3 (tear lipid layers extends slowly on not more than 1/2 of the surface); and G4 (tear lipid layers does not extend substantially).
   The tear volume on the ocular surface was measured by using the radius of tear meniscus curvature (R) and evaluated. Furthermore, to 35 cases 35 eyes (in total) classified as G2, G3 and G4 are exposed to the lacrimatory component (Thiopropanal S-oxide) and the influence of the exposure on the extensibility of the tear lipid layer was evaluated (the tear meniscus radius R and the extensibility Grade before and after exposure were compared).

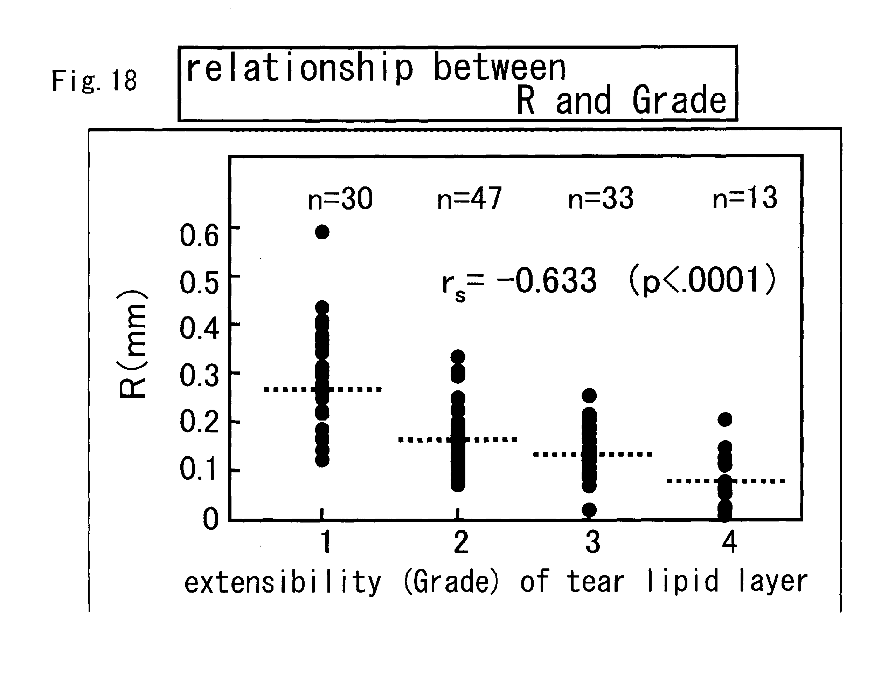

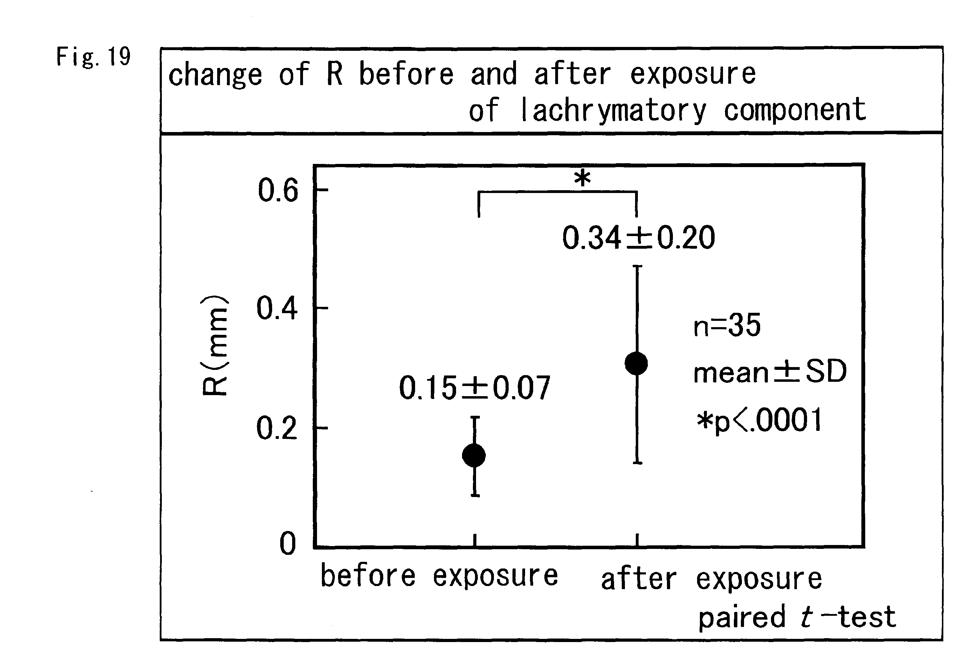

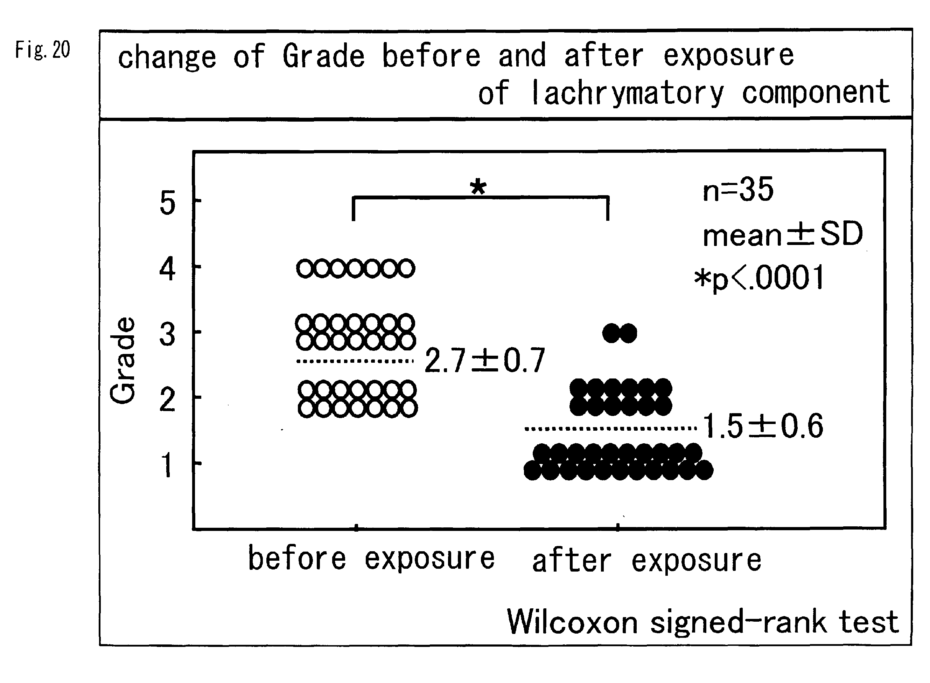

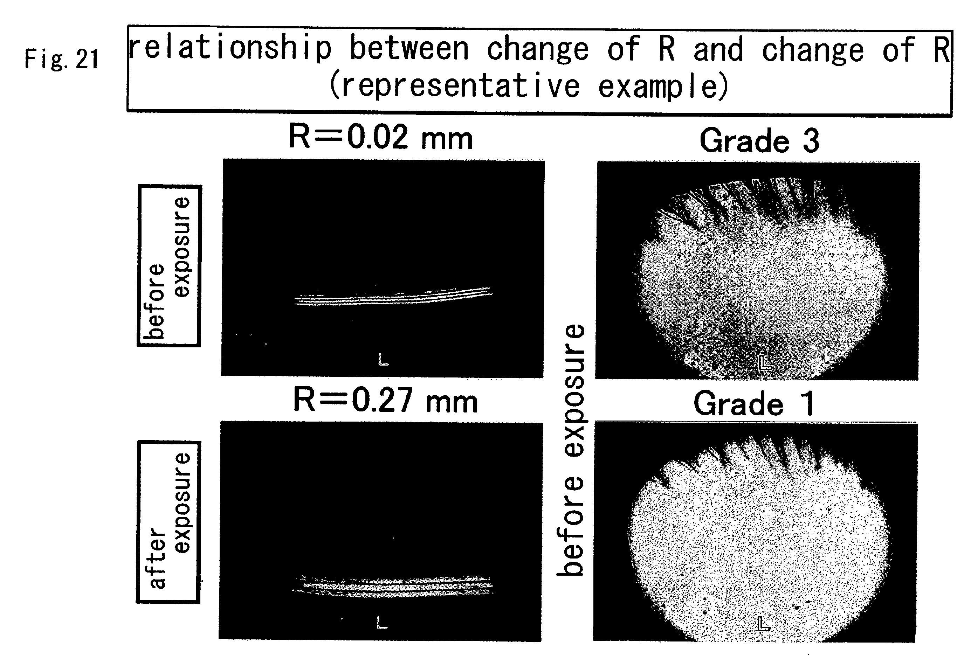

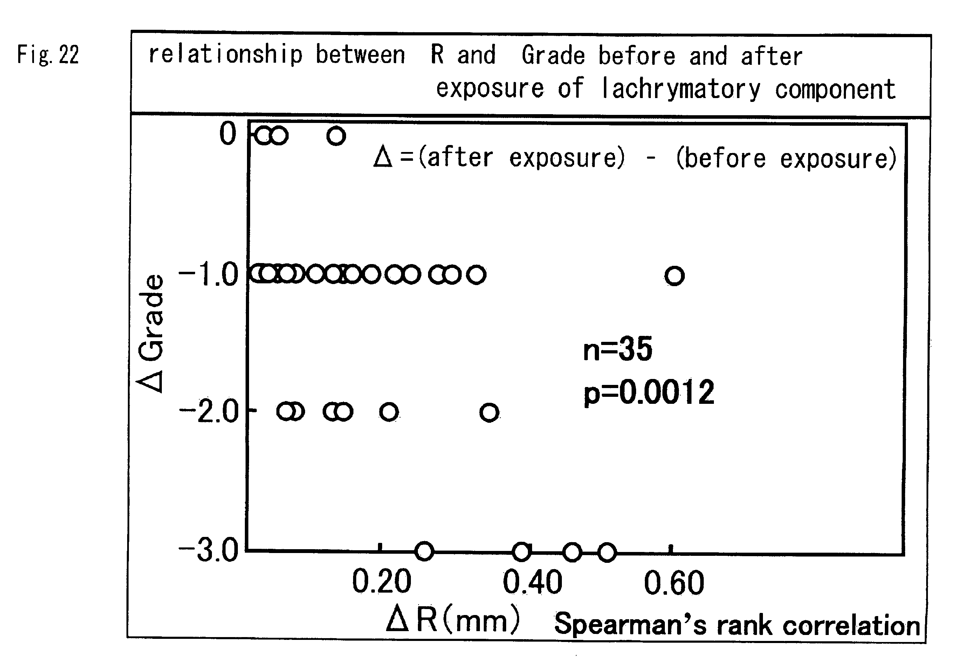

4. Results
   Rs (mm) were 0.273 ± 0.104 (mean value ± standard deviation) (G1, n=30), 0.171 ± 0.063 (G2, n=47), 0.135 ± 0.049 (G3, n=33), and 0.087±0.056 (G4, n=13), and decreased significantly with increasing extensibility grade (rₛ=-0.633, p<0.0001, Fig. 18). In 35 eyes which were exposed to the lacrimatory component, Rs increased from 0.15 ± 0.07 to 0.34 ±0.20 (mean value ± standard deviation, p<0.0001, paired t-test) (Fig. 19), and improvement in the extensibility of the tear lipid layer was observed from Grade 2.7 ± 0.7 to 1.5 ± 0.6 (mean value ± standard deviation, p<0.0001, Wilcoxon Matched-Pairs Signed-Ranks Test) (Fig. 20). Representative examples of R and Grade before and after the exposure of the lacrimatory component are shown in Fig. 21. Furthermore, Fig. 22 shows the relationship between the change in R and the change in the Grade due to the exposure of the lacrimatory component.

5. Conclusion
   Significant correlation between R and Grade was found. It was shown that the smaller the tear volume on the ocular surface was, the worse the extensibility of the tear lipid layer became. On the other hand, by exposing the eyes to the lacrimatory component, R increased significantly and the extensibility of the tear lipid layer improved (the extensibility Grade became smaller)., and the correlation between R and Grade was found significant. That is to say, it is shown that when the tear volume on the ocular surface is increased, the extensibility of the tear lipid layer can be improved.
   In the patients with tear deficient dry eye, the tear volume is small and the extensibility of the tear lipid layer is adversely affected under the influence of the tear volume. From the above-mentioned experimental results, it was revealed that when eyes of dry eye patients were exposed to the lacrimatory component of onions (Thiopropanal S-oxide), the tear volume was increased and the extension of the tear lipid layer was facilitated. This proves that the lacrimatory component is effective for treatment of dry eyes.

### [Example 5]

The relationship between the dynamics of tears on a soft contact lens (SCL) and the tear volume was examined. Unless otherwise specified, the exposure methods of a lacrimatory component and measurement methods of the tear volume, and the like, were the same as those in the above-mentioned Examples.

1. Purpose
   Tear layer covering the surface of a soft contact lens (SCL) is extremely thin and unstable. Therefore, the increase in the amount of water on the ocular surface may not be reflected immediately on the surface of the SCL worn for an extended period of time. Therefore, eyes of subjects who had been wearing SCL all day long were exposed to the lacrimatory component (Thiopropanal S-oxide) to induce the reflex lacrimation, and the change of the tear and the tear volume before and after the exposure were examined.

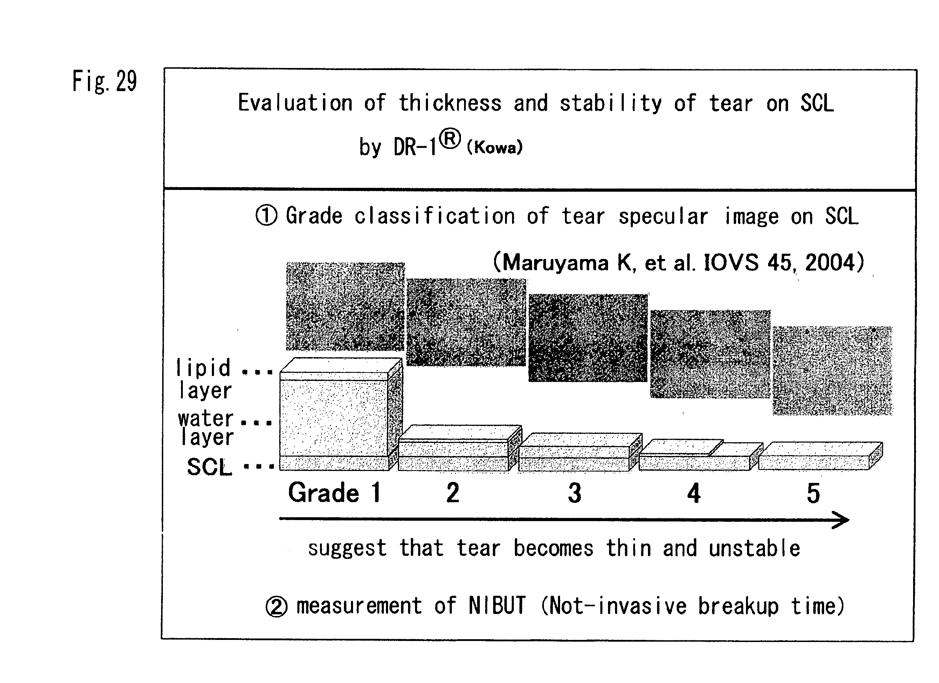

2. Subjects and method
   The subjects included 8 cases 8 eyes, regularly wearing SCL (male subjects, mean age: 21.4±1.6). The subjects were asked to wear SCL all day long and their eyes were exposed to the synthesized lacrimatory component of onions until the stimulation became no longer endurable. Then, before and after the exposure, the tear volume measurement (measurement of the radius of tear meniscus curvature by meniscometry: R) and observation of the dynamics of tears on SCL [observation of specular image of tears by interferometry (Grade classification 1 to 5: the higher the Grade is, the thinner the tear becomes) and measurement of Non-invasive break up time (NIBUT)] were carried out. The exposed eye was selected by an envelope method. Note here that Grade classifications of the specular image of tears on SCL are shown in Fig. 29.

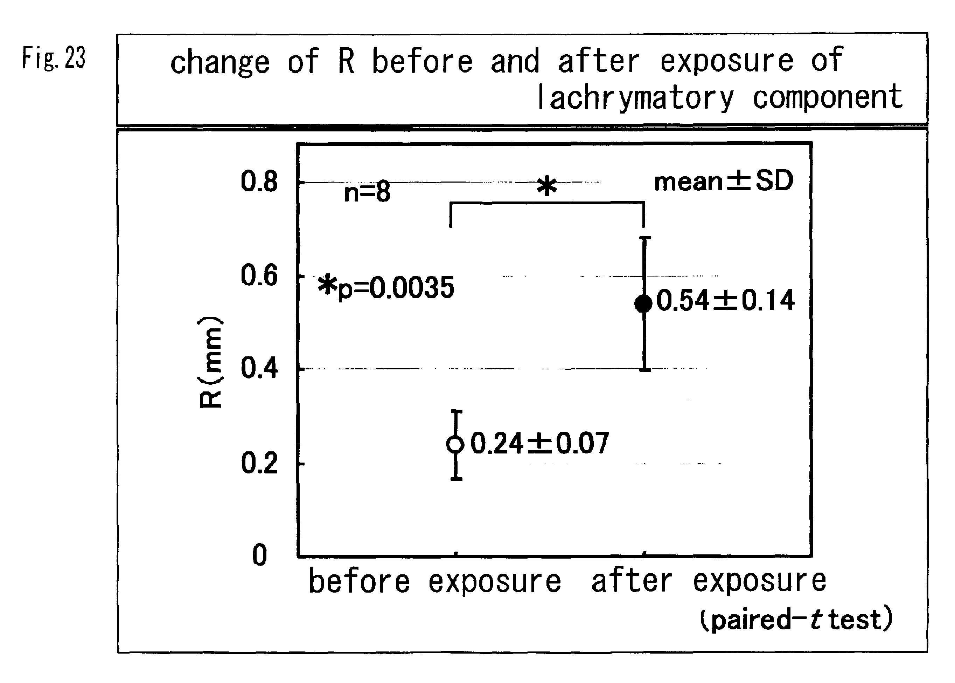

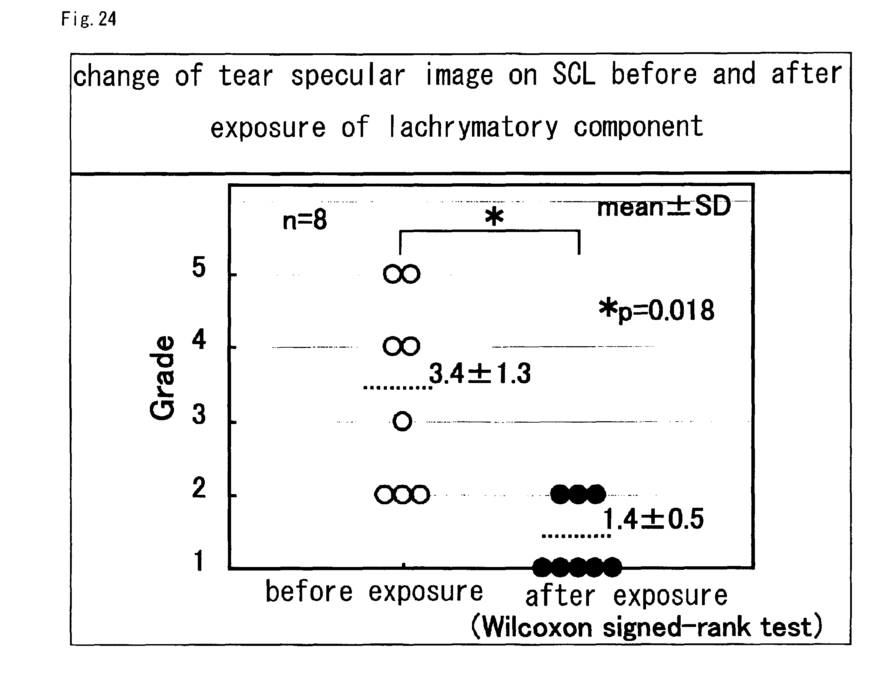

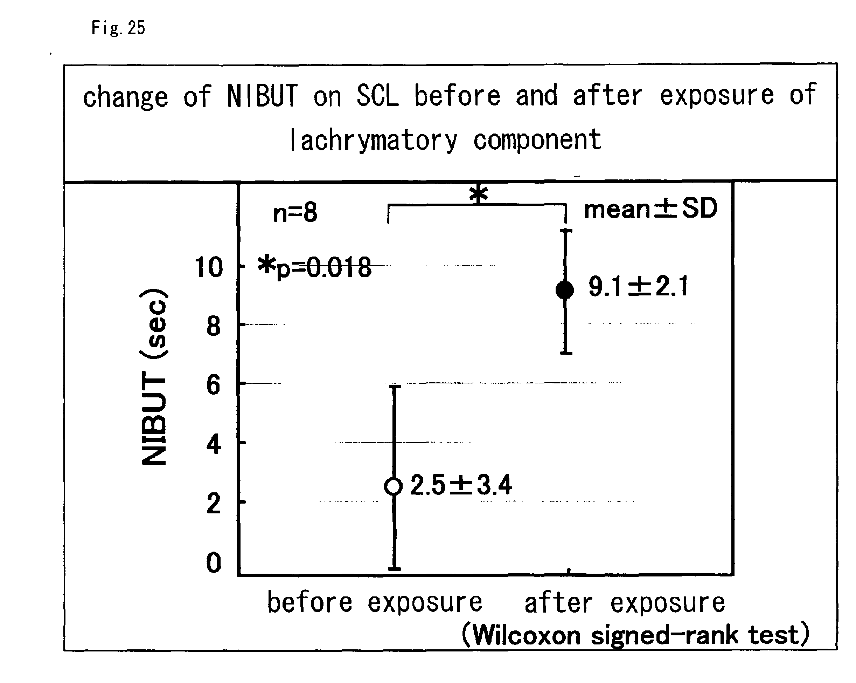

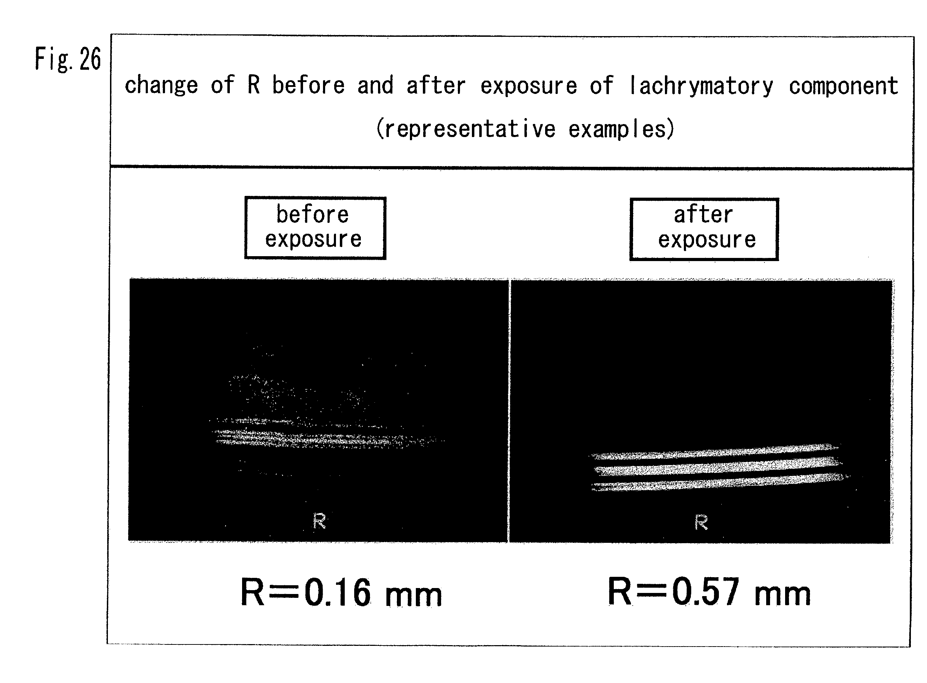

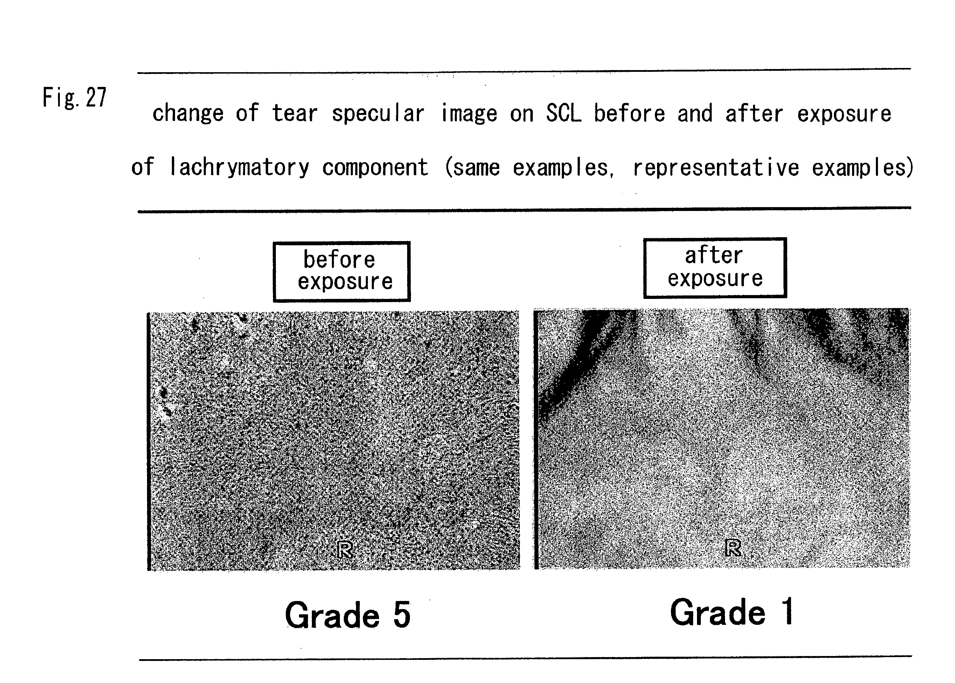

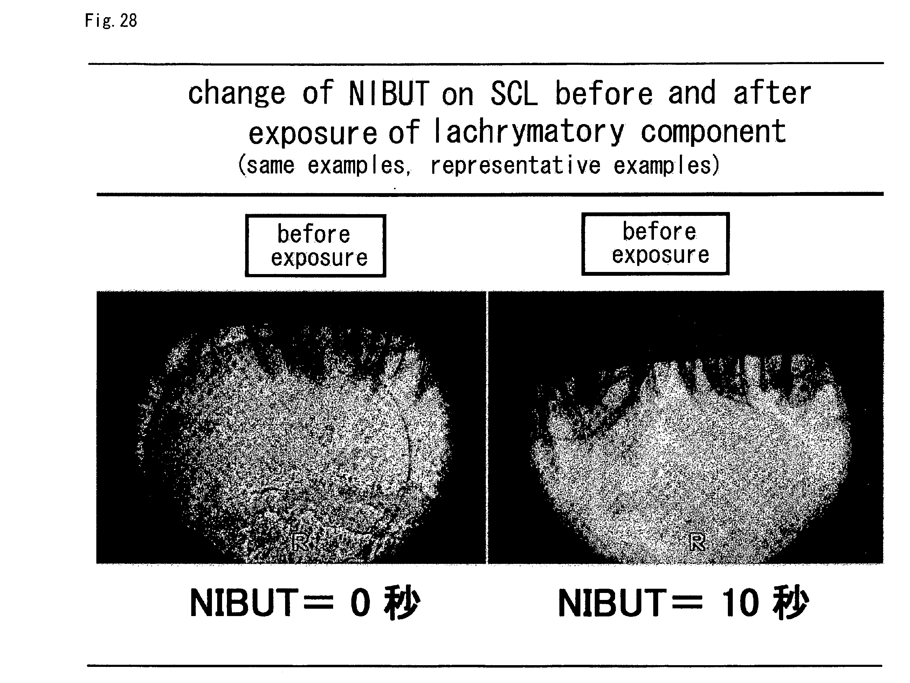

3. Results
   R (mm) increased from 0.24 ± 0.07 (mean value ± standard deviation) to 0.54 ± 0.14 (Fig. 23), and correspondingly the grades of specular image of the tears on SCL were significantly lowered from 3.4 ± 1.3 to 1.4 ± 0.5 (p<0.05, Wilcoxon Matched-Pairs Signed-Ranks Test, Fig. 24). NIBUT (in seconds) also became significantly longer from 2.5 ± 3.4 to 9.1 ± 2.1 (p<0.05, Wilcoxon Matched-Pairs Signed-Ranks Test, Fig. 25). Fig. 26 shows representative examples of R before and after the exposure to the lacrimatory component. Furthermore, Fig. 27 shows representative examples (the same examples) of specular images of tears on SCL before and after the exposure to the lacrimatory component. Furthermore, Fig. 28 shows representative examples (the same examples) of NIBUT of tear on SCL before and after the exposure of the lacrimatory component.

4. Conclusion
   The dynamics of tears on SCL was affected largely by the tear volume. When the tear volume increased, a tear layer on the lens is secured and the excellent extension of the lipid layer is obtained. It is shown that the stability of the tear on the lens can be improved.
   Furthermore, it was revealed that by the exposure to the lacrimatory component (Thiopropanal S-oxide), R was significantly increased; Grade of tear on SCL was significantly lowered; and NIBUT on SCL was significantly increased. These results show that the lacrimatory component has positive effects on (1) the increase of the tear volume in the subjects wearing SCL; (2) the increase of the thickness of tears on SCL; and (3) the increase of the stability of tears. That is to say, it is confirmed that the lacrimatory component is effective for treatment of dry eye associated with wearing of SCL.

### INDUSTRIAL APPLICABILITY

A reagent for lacrimation examination provided by the present invention can be used for examination of various diseases (for example, dry eye) in which the amount of lacrimation is a determining parameter. On the other hand, a lacrimation promoter composition provided by the present invention can be used as a therapeutic drug for various diseases (for example, dry eye) in which promoting lacrimation is effective in prevention or treatment of the diseases.

The present invention is not limited to the description of the above embodiments and Examples of the present invention. A variety of modifications, which are within the scopes of the claims and which can be easily achieved by a person skilled in the art, are included in the present invention.
All of the articles, patents and publications cited herein are hereby incorporated in their entirety by reference.

The following matters are disclosed.
1. A method of diagnosing dry eye, the method comprising:
   an exposure step of exposing an eye of a subject to the reagent for lacrimation examination described in any one of claims 1 to 3;
   a stimulation measurement step of measuring a length of time from the time when the exposure step is started to the time when the subject feels the stimulation (ST), or a degree of stimulation felt by the subject at a certain time point after the start of the exposure step; and
   a determination step of determining that the eye of the subject is dry eye when the ST is longer than a normal range of ST, or the degree of stimulation is lower than a normal range of stimulation.
2. A method of diagnosing dry eye, the method comprising:
   an initial tear volume measurement step of measuring a tear volume of a subject in an initial state (the initial tear volume);
   an exposure step of exposing an eye of the subject to the reagent for lacrimation examination described in any one of claims 1 to 3;
   a tear volume measurement step of measuring a tear volume of the subject at a certain time point after the exposure step(a post-exposure tear volume) and/or monitoring a increase with time of tear volume until that time point; and optionally,
   a calculation step of calculating the difference in the tear volume in the subject between the initial tear volume and the post-exposure tear volume; and
   a determination step of determining that the eye of the subject is dry eye when the tear volume increase is smaller than a normal range of the tear volume increase.
3. The method of diagnosing dry eye described in 2, wherein the increase with time of tear volume or the range of the tear volume increase is divided into a plurality of sections according to the degree of severity of dry eyes in advance, and by classifying the increase with time of tear volume or the range of the tear volume increase, the severity of dry eye is determined.
4. A method of diagnosing dry eye, the method comprising:
   an exposure step of exposing an eye of a subject to the reagent for lacrimation examination described in any one of claims 1 to 3;
   a tear volume measurement step of measuring a tear volume of the subject at a certain time point after the start of the exposure step; and
   a determination step of determining that the eye of the subject is dry eye when the obtained tear volume is smaller than a normal range of the tear volume.
5. The method of diagnosing dry eye described in any of 2 to 4, wherein the tear volume is measured using a radius of tear meniscus curvature.
6. A method of lacrimation examination described in any of 1 to 5, wherein the exposure of the reagent for lacrimation examination to the eye of the subject is accomplished in a non-contact manner.
7. A method of monitoring a degree of severity of dry eye, the method comprising:
   an exposure step of exposing an eye of a subject to the reagent for lacrimation examination described in any one of claims I to 3;
   a tear volume measurement step of measuring a tear volume of the subject at a certain time point after the start of the exposure step: and
   a determination step including: comparing the obtained tear volume with the tear volume that has been previously obtained by the similar procedure; and determining that a symptom is deteriorated when the tear volume is reduced and determining that a symptom is improved when the tear volume is increased.
8. A method of monitoring a degree of severity of dry eye, the method comprising:
   an initial tear volume measurement step of measuring a tear volume of a subject in an initial state (the initial tear volume);
   an exposure step of exposing an eye of a subject to the reagent for lacrimation examination described in any one of claims 1 to 3;
   a tear volume measurement step of measuring a tear volume of the subject at a certain time point after the exposure step (a post-exposure tear volume) ;
   a calculation step of calculating a the difference in the tear volume in the subject between the initial tear volume and the post-exposure tear volume; and
   a determination step including: comparing the calculated difference in the tear volume with the difference that was previously calculated by the similar procedure; and determining that a symptom is deteriorated when the difference is reduced and that a symptom is improved when the difference is increased.
9. The method of monitoring a degree of severity of dry eye described in 7 or 8, wherein the tear volume is measured using a radius of tear meniscus curvature.
10. The method of monitoring the degree of severity of dry eye described in any of 7 to 9, wherein the eye of a subject is exposed to the reagent for lacrimation examination in a non-contact manner.

11. A use of a compound for producing a lacrimation promoter composition, the compound is represented by the following chemical formula: 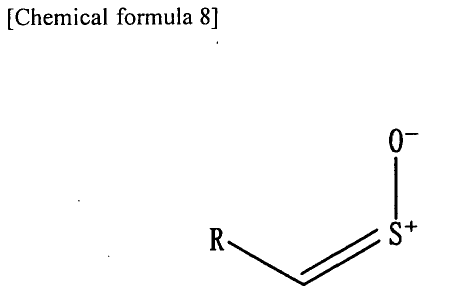 wherein R represents a C₁₋₅ alkyl chain.
12. A use of a compound for producing a lacrimation promoter composition, the compound is represented by the following chemical formula. 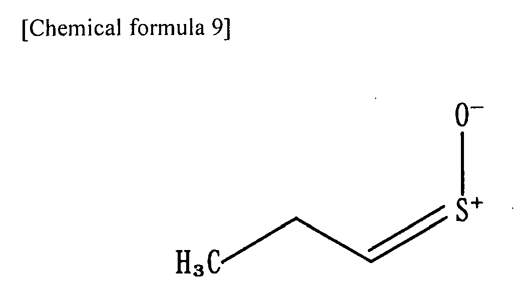
13. The use described in 11 or 12, wherein the lacrimation promoter composition is used for prevention or treatment of dry eye.

## Claims

1. A reagent for lacrimation examination, comprising as an active ingredient a compound represented by the following chemical formula: 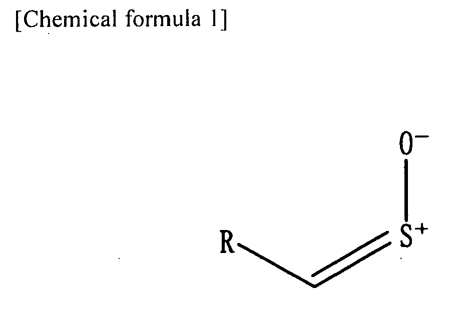 wherein R represents a C₁₋₅ alkyl chain.

2. A reagent for lacrimation examination, comprising as an active ingredient a compound represented by the following chemical formula.

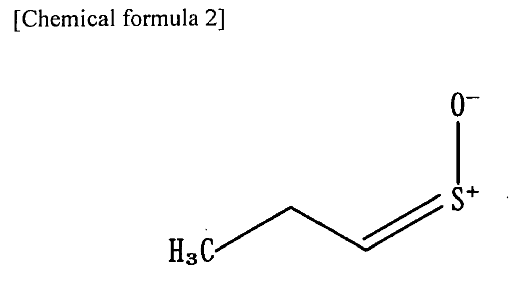

3. The reagent for lacrimation examination according to claim 1, which is used for examining dry eye.

4. A lacrimation examination kit, comprising:
the reagent for lacrimation examination according to claim 1; and
an exposure container used for exposing the eye to the reagent for lacrimation examination, the exposure container being used to cover the eye in a state in which the inner side thereof faces the eye after the reagent for lacrimation examination is placed in the exposure container.

5. A method of lacrimation examination, the method comprising:
exposing an eye of a subject to the reagent for lacrimation examination according to claim 1.

6. A method of lacrimation examination, the method comprising:
an exposure step of exposing an eye of a subject to the reagent for lacrimation examination according to claim 1; and
a stimulation measurement step of measuring a length of time from the time when the exposure step is started to the time when the subject feels the stimulation, or a degree of stimulation felt by the subject at a certain time point after the start of the exposure step.

7. A method of lacrimation examination, the method comprising:
an initial tear volume measurement step of measuring a tear volume of a subject in an initial state (the initial tear volume);
an exposure step of exposing an eye of the subject to the reagent for lacrimation examination according to claim 1;
a tear volume measurement step of measuring a tear volume of the subject at a certain time point after the exposure step(a post-exposure tear volume) and/or monitoring a increase with time of the tear volume until that time point; and optionally,
a calculation step of calculating the difference in the tear volume in the subject between the initial tear volume and the post-exposure tear volume.

8. A method of lacrimation examination, the method comprising:
an exposure step of exposing an eye of a subject to the reagent for lacrimation examination according to claim 1; and
a tear volume measurement step of measuring a tear volume of the subject at a certain time point after the exposure step.

9. The method of lacrimation examination according to claim 7, wherein the tear volume is measured using a radius of tear meniscus curvature.

10. The method of lacrimation examination according to claim 5, wherein the exposure of the reagent for lacrimation examination to the eye of the subject is accomplished in a non-contact manner.

11. A lacrimation promoter composition, containing as an active ingredient a compound represented by the following chemical formula: 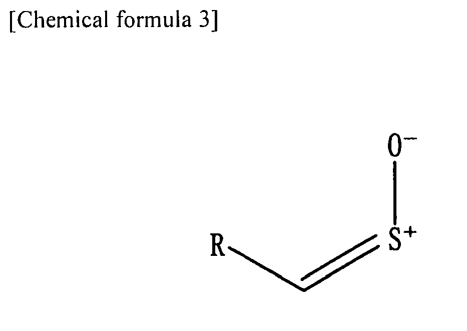 wherein R represents a C₁₋₅ alkyl chain.

12. A lacrimation promoter composition, containing as an active ingredient a compound represented by the following chemical formula.

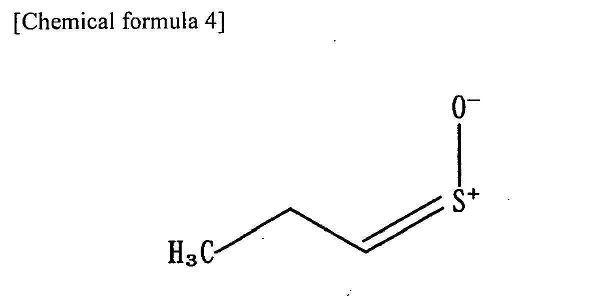

13. The lacrimation promoter composition according to claim 11, which is used for prevention or treatment of dry eye.

14. A lacrimation promoter kit, comprising:
the lacrimation promoter composition according to claim 11; and
an exposure container used for exposing an eye to the reagent for lacrimation examination, wherein the exposure container is used to cover the eye in a state in which the inner side thereof faces the eye after the lacrimation promoter composition is placed in the exposure container.
